# EUROPEAN PATENT APPLICATION

(11) **EP 4 389 897 A1**
(43) Date of publication of application: **26.06.2024**
(21) Application number: 22858543.6
(22) Date of filing: 19.08.2022
(51) Int. Cl.: C12N 15/55, A01H 1/00, C12N 15/09, C12N 15/83

(54) **POLYNUCLEOTIDE INCLUDING SITE-SPECIFIC NUCLEASE EXPRESSION CASSETTE**

(30) Priority: 20.08.2021 JP 2021134908
(71) Applicant: GRA&GREEN Inc., Nagoya-shi, Aichi 464-0807 (JP)
(72) Inventor: NIWA, Masaki, Nagoya-shi, Aichi 464-0814 (JP); KOBAYASHI, Takehito, Nagoya-shi, Aichi 464-0814 (JP); SAWAI, Yu, Nagoya-shi, Aichi 464-0814 (JP); OKUDA, Atsushi, Nagoya-shi, Aichi 464-0814 (JP)
(74) Representative: Hoffmann Eitle
(86) International application number: PCT/JP2022/031362
(87) International publication number: WO 2023/022226

(57) **Abstract**

An object is to provide a plant genome editing technique with higher genome editing efficiency. This object is achieved by a polynucleotide comprising a site-specific nuclease expression cassette and a sequence that binds to a nuclear translocation factor and/or an intranuclear structure, the site-specific nuclease expression cassette comprising (b) a site-specific nuclease coding sequence and (c) a 3'UTR comprising a terminator.

## Description

### Technical Field

The present invention relates to a polynucleotide comprising a site-specific nuclease expression cassette and the like.

### Background Art

In plants, traditional breeding, molecular breeding, genetic modification, and the like have been used to improve or modify plants. They can, for example, improve or modify plant traits such as yield, environmental adaptability, disease resistance, insect resistance, and growth rate.

In recent years, genome editing techniques using a site-specific nuclease have attracted attention as new plant breeding techniques. The CRISPR/Cas system is known as one of such techniques. Typically, target genes can be mutated in various animals and plants by introducing Cas protein and guide RNA.

In genome editing techniques, improvement in their efficiency is desired. In particular, in the case of mutating a plurality of genes at the same time, if the efficiency is low, it is not possible to introduce mutations into all of the desired genes at the same time.

Patent Literature (PTL) 1 discloses a technique to improve expression by placing a terminator and a scaffold/matrix attachment region in an expression cassette. However, PTL 1 does not disclose or suggest expressing a site-specific nuclease.

### Citation List

### Patent Literature

PTL 1: US Patent Application Publication No. 2020/0407741

### Summary of Invention

### Technical Problem

An object of the present invention is to provide a plant genome editing technique with higher genome editing efficiency.

### Solution to Problem

The present inventors conducted extensive research to achieve the above object, and found that the object can be achieved by a polynucleotide comprising a site-specific nuclease expression cassette and a sequence that binds to a nuclear translocation factor and/or an intranuclear structure, the site-specific nuclease expression cassette comprising (b) a site-specific nuclease coding sequence and (c) a 3'UTR comprising a terminator. The inventors conducted further research on the basis of this finding and completed the present invention. Specifically, the present invention includes the following subject matter.

Item 1. A polynucleotide comprising a site-specific nuclease expression cassette and a sequence that binds to a nuclear translocation factor and/or an intranuclear structure, the site-specific nuclease expression cassette comprising:
(b) a site-specific nuclease sequence; and
(c) a 3'UTR comprising a terminator.

Item 2. The polynucleotide according to Item 1, wherein the sequence that binds to a nuclear translocation factor and/or an intranuclear structure is an insulator.

Item 3. The polynucleotide according to Item 1 or 2, wherein the sequence that binds to a nuclear translocation factor and/or an intranuclear structure is contained in the 3'UTR.

Item 4. The polynucleotide according to any one of Items 1 to 3, wherein the sequence that binds to a nuclear translocation factor and/or an intranuclear structure is an insulator other than a scaffold/matrix attachment region.

Item 5. The polynucleotide according to Item 4, wherein the insulator is at least one member selected from the group consisting of an Ars insulator, a Gypsy insulator, a Bead1 insulator, a TEF insulator, and a TEF2 insulator.

Item 6. The polynucleotide according to Item 5, wherein the insulator is an Ars insulator.

Item 7. The polynucleotide according to any one of Items 1 to 3, wherein the sequence that binds to a nuclear translocation factor and/or an intranuclear structure is a scaffold/matrix attachment region.

Item 8. The polynucleotide according to any one of Items 1 to 7, wherein the terminator is a linked body of at least two terminators.

Item 9. The polynucleotide according to any one of Items 1 to 8, wherein the sequence that binds to a nuclear translocation factor and/or an intranuclear structure is located in the 3'UTR and downstream of the terminator.

Item 10. The polynucleotide according to any one of Items 1 to 9, wherein the terminator is at least one member selected from the group consisting of a 35S terminator, an ACT3 terminator, and an extensin terminator.

Item 11. The polynucleotide according to any one of Items 1 to 10, wherein the site-specific nuclease expression cassette comprises (a) a promoter.

Item 12. The polynucleotide according to Item 11, wherein the promoter is a shoot apex expression promoter.

Item 13. The polynucleotide according to Item 11 or 12, wherein the promoter is a CmYLCV promoter, a UBQ promoter, an EF-1α promoter, or an RPS5A promoter.

Item 14. The polynucleotide according to any one of Items 1 to 13, which comprises a virus vector sequence.

Item 15. The polynucleotide according to Item 14, wherein the virus vector sequence is a geminivirus vector sequence.

Item 16. The polynucleotide according to Item 14 or 15, wherein the virus vector sequence is a BeYDV sequence.

Item 17. The polynucleotide according to any one of Items 1 to 16, further comprising a guide RNA expression cassette.

Item 18. A vector for plant genome editing, comprising the polynucleotide of any one of Items 1 to 17.

Item 19. A kit for plant genome editing, comprising the polynucleotide of any one of Items 1 to 17.

Item 20. A plant genome editing method comprising introducing the polynucleotide of any one of Items 1 to 17 into a plant.

Item 21. The plant genome editing method according to Item 20, further comprising introducing a mitotic cell activating factor into the site of introduction of the polynucleotide.

Item 22. A method for producing a genome-edited plant, comprising introducing the polynucleotide of any one of Items 1 to 17 into a plant.

Item A. A composition for plant genome editing, comprising a polynucleotide that comprises a mitotic cell activating factor expression cassette.

Item B. An agent for improving plant genome editing efficiency, comprising a polynucleotide that comprises a mitotic cell activating factor expression cassette.

Item C. A plant genome editing method or method for producing a genome-edited plant, comprising introducing a site-specific nuclease expression cassette and a mitotic cell activating factor expression cassette into a plant.

Item D. A method for improving plant genome editing efficiency, comprising introducing a mitotic cell activating factor expression cassette into a plant.

### Advantageous Effects of Invention

The present invention provides a plant genome editing technique with higher genome editing efficiency.

### Brief Description of Drawings

Fig. 1 shows a vector map of the Cas expression vector constructed in Example 1. Arrows indicate promoters.
Fig. 2 shows a vector map of the Cas expression vectors constructed in Comparative Examples 1 and 2. Arrows indicate promoters.
Fig. 3 shows the results (genome editing efficiency) of Test Example 1. The upper end of the bar above each box indicates the maximum value, the upper end of each box indicates the third quartile, the horizontal line in each box indicates the median, the symbol "×" in each box indicates the mean value, the lower end of each box indicates the first quartile, and the lower end of the bar below each box indicates the minimum value. Gray circles indicate outliers.
Fig. 4 shows the results of Test Example 2-2. Fig. 4(a) shows a chromatogram containing a target sequence obtained by Sanger sequencing, and Fig. 4(b) shows the results of mutation analysis by the analysis tool "DECODR."
Fig. 5 shows the results of Test Example 2-3. Fig. 5(a) and Fig. 5(b) show the results obtained by using different guide RNAs. Uncleaved indicates an uncleaved band, WT indicates the case in which no Cas expression vector was introduced, and #33 indicates the case in which a Cas expression vector was introduced. Genome editing efficiency is shown below the lanes.
Fig. 6 shows a vector map of the Cas expression vector constructed in Example 4. Arrows indicate promoters.
Fig. 7 shows a vector map of the Cas expression vectors constructed in Comparative Example 4 and Examples 3 and 5 to 7. Arrows indicate promoters.
Fig. 8 shows the results (genome editing efficiency) of Test Example 3. The upper end of the bar above each box indicates the maximum value, the upper end of each box indicates the third quartile, the horizontal line in each box indicates the median, the symbol "×" in each box indicates the mean value, the lower end of each box indicates the first quartile, and the lower end of the bar below each box indicates the minimum value. NtEU indicates Comparative Example 4, and NtEU_Ars indicates Example 4.
Fig. 9 shows the results of Test Example 4. Uncleaved indicates an uncleaved band. MAD7 indicates the case in which the effector expression vector of Example 8 was introduced, and WT indicates the case in which no vector was introduced. Genome editing efficiency is shown below the lanes.

### Description of Embodiments

### 1. Definition

In the present specification, the terms "comprising," "containing," "including," and "having" include the concepts of containing, including, consisting essentially of, and consisting of.

In the present specification, the "identity" of amino acid sequences refers to the degree of identicalness of two or more amino acid sequences that can be compared with each other. Thus, the higher the identicalness of two amino acid sequences, the higher the identity or similarity of these sequences. The level of amino acid sequence identity is determined by, for example, using FASTA, which is a tool for sequence analysis, with default parameters. The level of amino acid sequence identity can otherwise be determined by using the algorithm BLAST by Karlin and Altschul (Karlin S, Altschul SF, "Methods for assessing the statistical significance of molecular sequence features by using general scoring schemes," Proc Natl Acad Sci USA, 87: 2264-2268 (1990); Karlin S, Altschul SF, "Applications and statistics for multiple high-scoring segments in molecular sequences," Proc Natl Acad Sci USA, 90: 5873-7 (1993)). Programs called "blastp" and "tblastn," which are based on such an algorithm of BLAST, have been developed. Specific procedures for these analysis methods are known, and reference may be made to the National Center of Biotechnology Information (NCBI) website (http://www.ncbi.nlm.nih.gov/). The "identity" of base sequences is also defined accordingly.

In the present specification, "conservative substitution" means a substitution of an amino acid residue with an amino acid residue having a similar side chain. For example, a substitution between amino acid residues having a basic side chain, such as lysine, arginine, and histidine, is considered to be a conservative substitution. Other examples that are considered to be a conservative substitution include a substitution between amino acid residues having an acidic side chain, such as aspartic acid and glutamic acid; a substitution between amino acid residues having an uncharged polar side chain, such as asparagine, glutamine, serine, threonine, tyrosine, and cysteine; a substitution between amino acid residues having a nonpolar side chain, such as glycine, alanine, valine, leucine, isoleucine, proline, phenylalanine, methionine, and tryptophan; a substitution between amino acid residues having a branched chain, such as valine, isoleucine, and leucine; and a substitution between amino acid residues having an aromatic side chain, such as tyrosine, phenylalanine, and tryptophan.

In the present specification, the "coding sequence" is not particularly limited as long as it is a base sequence encoding the amino acid sequence of a protein.

In the present specification, the 5' side means the 5' side of the sense strand of the site-specific nuclease coding sequence, and the 3' side means the 3' side of the sense strand of the site-specific nuclease coding sequence. The 5' side may be referred to as "upstream," and the 3' side may be referred to as "downstream."

In the present specification, the "plant" is not particularly limited. Examples of plants include a wide range of plants, including bryophytes, pteridophytes, gymnosperms, magnoliids of angiosperms, monocots, and eudicots (eurosid I, eurosid II, euasterids I, euasterids II, and their out-groups). More specific examples of plants include *Solanum melongena* plants, such as tomatoes, green bell peppers, *Capsicum annuum,* eggplants, *Nicotiana tabacum,* and *Solanum torvum; Cucurbitaceae* plants, such as cucumber, *Cucurbita* plants, melon, and watermelon; *Brassica rapa* plants, such as cabbage, broccoli, and napa cabbage; green leaf and spicy leaf vegetables, such as celery, parsley, and lettuce; *Allium fistulosum* plants, such as Welsh onions, onions, and garlic; other fruit and vegetable crops, such as strawberries and melons; taproot plants, such as *daikon radish, white turnip,* carrot, and greater burdock; roots and tubers, such as taros, cassava, potatoes, white potatoes, sweet potatoes, and Chinese yam; grains, such as rice, corn, wheat, sorghum, barley, rye, purple false brome, and buckwheat; beans, such as soybeans, adzuki beans, mung beans, black-eyed beans, *Phaseolus vulgaris,* peanuts, peas, and *Vicia faba;* soft vegetables, such as asparagus, spinach, and *Cryptotaenia japonica;* flower plants, such as *Eustoma grandiflorum, Matthiola incana, Dianthus caryophyllus,* and *Chrysanthemum morifolium;* grass, such as *Agrostis stolonifera* and *Zoysia matrella;* oil crops, such as rapeseed, peanuts, *Brassica napus, Jatropha curcas,* sesame, and *Perilla frutescens;* fiber crops, such as cotton and *Juncus effusus;* feed crops, such as clover, dent corn, and *Medicago truncatula;* deciduous fruit trees, such as apples, pears, grapes, and peaches; citrus fruits, such as *Citrus unshiu,* oranges, lemons, and grapefruits; woody plants, such as *Rhododendron indicum, Rhododendron* plants, *Cryptomeria, Populus,* and *Hevea brasiliensis;* and the like.

### 2. Polynucleotide

In one embodiment, the present invention relates to a polynucleotide comprising a site-specific nuclease expression cassette and a sequence that binds to a nuclear translocation factor and/or an intranuclear structure, the site-specific nuclease expression cassette comprising (b) a site-specific nuclease coding sequence and (c) a 3'UTR comprising a terminator (which may be referred to as "the polynucleotide of the present invention" in the present specification). The polynucleotide of the present invention is described below.

The polynucleotide of the present invention comprises a site-specific nuclease expression cassette. The site-specific nuclease expression cassette is not particularly limited as long as it comprises a base sequence necessary to express a site-specific nuclease, specifically, to transcribe mRNA capable of producing a site-specific nuclease.

In the polynucleotide of the present invention, the site-specific nuclease expression cassette comprises (b) a site-specific nuclease coding sequence. The site-specific nuclease coding sequence is not particularly limited as long as it is a base sequence encoding the amino acid sequence of a site-specific nuclease.

The site-specific nuclease is not particularly limited as long as it is a nuclease capable of specifically cleaving a target site on genomic DNA to generate cleavage fragments. Examples of site-specific nucleases include Cas proteins, zinc finger nucleases (ZFN), TAL effector nucleases (TALEN), and the like. ZFN is a fusion protein of several zinc finger motifs that recognize specific bases and a DNA cleavage effector domain. TALEN is a fusion protein of a transcription activator-like (TAL) effector and a DNA cleavage effector domain. The site-specific nuclease may be composed of another additional targeting technique such as a meganuclease or a leucine zipper. The site-specific nuclease is particularly preferably a Cas protein.

Cas proteins are not particularly limited as long as they are those that are used in the CRISPR/Cas system. For example, various types of Cas proteins that can bind to and cleave a target site of genomic DNA while forming a complex with guide RNA can be used. Known Cas proteins include those from various organisms. Examples include a Cas9 protein (type II) from *S. pyogenes,* a Cas protein (type I-A) from *S. solfataricus,* a Cas protein (type I-A) from *S. solfataricus,* a Cas protein (type I-B) from *H. walsbyi,* a Cas protein (type I-D) from *Microcystis aeruginosa,* a Cas protein (type I-E) from E. *coli,* a Cas protein (type I-F) from E. *coli,* a Cas protein (type I-F) from P. *aeruginosa,* a Cas9 protein (type II) from *S. thermophilus,* a Cas9 protein (type II) from *S. agalactiae,* a Cas9 protein from *S. aureus,* a Cas9 protein from *N. meningitidis,* a Cas9 protein from *T. denticola,* a Cpf1 protein (type V-A) from *F. novicida,* a MAD7 protein (type V-A) from *Eubacterium rectale,* and the like. Of these, preferred examples include Cas9 proteins, and more preferred examples include Cas9 proteins endogenously found in bacteria belonging to the genus *Streptococcus.* The information on the amino acid sequences of various Cas proteins and their coding sequences can be easily obtained from various databases, such as NCBI.

The Cas protein may be a wild-type double-strand break-generating Cas protein or a Nickase Cas protein. The double-strand break-generating Cas protein usually comprises a domain involved in cleavage of target strand (HNH domain) and a domain involved in cleavage of non-target strand (RuvC domain). The Nickase Cas protein may be, for example, a protein comprising a mutation that reduces the cleavage activity of one of the two domains of the double-strand break-generating Cas protein (e.g., reduces the cleavage activity to 1/2, 1/5, 1/10, 1/100, or 1/1000 or less). For example, if the double-strand break-generating Cas protein is a Cas9 protein from *S. pyogenes,* examples of such a mutation include a mutation of the 10th amino acid (aspartic acid) from the N-terminus to alanine (D10A: a mutation in the RuvCI domain), a mutation of the 840th amino acid (histidine) from the N-terminus to alanine (H840A: a mutation in the HNH domain), a mutation of the 863rd amino acid (asparagine) from the N-terminus to alanine (N863A: a mutation in the HNH domain), a mutation of the 762nd amino acid (glutamic acid) from the N-terminus to alanine (E762A: a mutation in the RuvCII domain), a mutation of the 986th amino acid (aspartic acid) from the N-terminus to alanine (D986A: a mutation in the RuvCIII domain), and the like.

The Cas protein may comprise one or more amino acid sequence mutations (e.g., substitution, deletion, insertion, and addition) as long as the activity is not impaired. From this perspective, the Cas protein may be a protein comprising an amino acid sequence having, for example, 85% or more, preferably 90% or more, more preferably 95% or more, and even more preferably 98% or more identity to the amino acid sequence of a wild-type double-strand break-generating Cas protein or a Nickase Cas protein based on the wild-type double-strand break-generating Cas protein, and having the activity thereof (the activity to bind to and cleave a target site of genomic DNA while forming a complex with guide RNA). Alternatively, from the same perspective, the Cas protein may be a protein comprising an amino acid sequence in which one or more (e.g., 2 to 100, preferably 2 to 50, more preferably 2 to 20, even more preferably 2 to 10, still more preferably 2 to 5, and particularly preferably 2) amino acids are substituted, deleted, added, or inserted (preferably conservatively substituted) in the amino acid sequence of a wild-type double-strand break-generating Cas protein or a Nickase Cas protein based on the wild-type double-strand break-generating Cas protein, and having the activity thereof (the activity to bind to and cleave a target site of genomic DNA while forming a complex with guide RNA). The above "activity" can be evaluated in vitro or in vivo in accordance with a known method or an equivalent method.

The site-specific nuclease may be one to which a known protein tag, a signal sequence, or a protein such as an enzyme protein is added as long as it has the "activity" described above. Examples of protein tags include biotin, a His tag, a FLAG tag, a Halo tag, an MBP tag, an HA tag, a Myc tag, a V5 tag, a PA tag, and the like. Examples of signal sequences include nuclear localization signals and the like. Examples of enzyme proteins include various histone-modifying enzymes, deaminases, and the like.

In the polynucleotide of the present invention, the site-specific nuclease expression cassette comprises (c) a 3'UTR comprising a terminator. The 3'UTR is located on the 3' side of the site-specific nuclease coding sequence.

The terminator is not particularly limited as long as it is a base sequence that terminates transcription of DNA into mRNA.

Preferably, the terminator may be a terminator derived from a plant gene or a plant virus. The terminator is located on the 3' side of the coding region of a gene and can be easily identified by sequence analysis. Preferred examples of the terminator include a 35S (preferably cauliflower mosaic virus 35S) terminator (specifically, for example, SEQ ID NO: 4 or a mutant sequence thereof), an ACT3 (preferably *Nicotiana benthamiana* ACT3) terminator (specifically, for example, SEQ ID NO: 5 or a mutant sequence thereof), an extensin (preferably *Nicotiana tabacum* extensin) terminator (specifically, for example, SEQ ID NO: 14 or a mutant sequence thereof), and the like.

The terminator may comprise one or more base sequence mutations (e.g., substitution, deletion, insertion, and addition) as long as the function is not significantly reduced. The terminator has, for example, 70% or more, preferably 80% or more, more preferably 90% or more, even more preferably 95% or more, still even more preferably 97% or more, and particularly preferably 99% or more identity to the base sequence of a wild-type terminator. The terminator also includes those that have been modified to enhance the function of the terminator, for example, by linking a plurality of elements or by increasing the length in bases of a specific sequence.

The presence or absence of the function of the terminator can be determined by expressing mRNA from an expression cassette comprising the target base sequence in the 3'UTR and using an index such that the amount of the mRNA produced when the target base sequence functions as a terminator (which can be determined by the base length) is, for example, 50 mol% or more, preferably 70 mol% or more, more preferably 80 mol% or more, and even more preferably 90 mol% or more, based on the total mRNA produced from the expression cassette taken as 100 mol%.

The terminator is preferably, for example, a terminator comprising a base sequence with 70% or more identity to the base sequence set forth in SEQ ID NO: 4, a terminator comprising a base sequence with 70% or more identity to the base sequence set forth in SEQ ID NO: 5, or a terminator comprising a base sequence with 70% or more identity to the base sequence set forth in SEQ ID NO: 14.

As the terminator, only one terminator may be used, or a plurality of terminators may be used. In a preferred embodiment of the present invention, the terminator is a linked body of at least two terminators. In the linked body, the terminators may be directly linked or indirectly linked via another sequence. When a plurality of terminators are used, the plurality of terminators may be the same terminator or different terminators.

The polynucleotide of the present invention comprises a sequence that binds to a nuclear translocation factor and/or an intranuclear structure. The sequence that binds to a nuclear translocation factor and/or an intranuclear structure may be located anywhere in the polynucleotide of the present invention. The sequence that binds to a nuclear translocation factor and/or an intranuclear structure is preferably located in the site-specific nuclease expression cassette, more preferably in the 3'UTR of the expression cassette, and particularly preferably in the 3'UTR and downstream of the terminator.

The sequence that binds to a nuclear translocation factor and/or an intranuclear structure is preferably, for example, an insulator.

The insulator is not particularly limited as long as it is a base sequence that can separate a transcriptional regulatory mechanism even between adjacent genes.

The insulator is, for example, a scaffold/matrix attachment region. The scaffold/matrix attachment region is a region to which the nuclear matrix attaches and includes the function of delimiting individual chromosomal regions by inducing chromatin loop formation.

Preferably, the scaffold/matrix attachment region may be a scaffold/matrix attachment region of a plant. The scaffold/matrix attachment region can be easily identified by known information and sequence analysis. The scaffold/matrix attachment region is preferably, for example, an Rb7 (preferably *Nicotiana tabacum* Rb7) scaffold/matrix attachment region (specifically, for example, SEQ ID NO: 6 or a mutant sequence thereof).

The scaffold/matrix attachment region may comprise one or more base sequence mutations (e.g., substitution, deletion, insertion, and addition) as long as the function is not significantly reduced. The scaffold/matrix attachment region has, for example, 70% or more, preferably 80% or more, more preferably 90% or more, even more preferably 95% or more, still even more preferably 97% or more, and particularly preferably 99% or more identity to the base sequence of a wild-type scaffold/matrix attachment region. The scaffold/matrix attachment region also includes those that have been modified to enhance the function of the scaffold/matrix attachment region, for example, by linking a plurality of elements or by increasing the length in bases of a specific sequence.

The scaffold/matrix attachment region is preferably, for example, a scaffold/matrix attachment region comprising a base sequence with 70% or more identity to the base sequence set forth in SEQ ID NO: 6.

Examples of insulators other than scaffold/matrix attachment regions include enhancer-blocking insulators that have the function of forming a chromatin loop domain in the nucleus and separating an enhancer and a target gene promoter, barrier insulators that inhibit a specific process in the heterochromatin formation pathway, and the like. The insulator other than scaffold/matrix attachment regions can be easily identified by known information and sequence analysis. Preferred examples of the insulator other than scaffold/matrix attachment regions include a *Drosophila* Gypsy insulator (SEQ ID NO: 15 or a mutant sequence thereof), a human Bead1 (blocking element alpha/delta-1 of the human T-cell receptor α/ δ locus) insulator (SEQ ID NO: 16 or a mutant sequence thereof), a sea urchin Ars (arylsulfatase) insulator (SEQ ID NO: 17 or a mutant sequence thereof), an A. *gossypii* TEF (translation elongation factor) insulator (SEQ ID NO: 18 or a mutant sequence thereof), and a yeast TEF2 (translation elongation factor 2) insulator (SEQ ID NO: 19 or a mutant sequence thereof). Among these, an Ars insulator is particularly preferable from the viewpoint of further significantly improving genome editing efficiency.

The insulator other than scaffold/matrix attachment regions may comprise one or more base sequence mutations (e.g., substitution, deletion, insertion, and addition) as long as the function is not significantly reduced. The insulator other than scaffold/matrix attachment regions has, for example, 70% or more, preferably 80% or more, more preferably 90% or more, even more preferably 95% or more, still even more preferably 97% or more, and particularly preferably 99% or more identity to the base sequence of a wild-type insulator other than scaffold/matrix attachment regions. The insulator other than scaffold/matrix attachment regions also includes those that have been modified to enhance the function thereof, for example, by linking a plurality of elements or by increasing the length in bases of a specific sequence.

Preferred examples of the insulator other than scaffold/matrix attachment regions include an insulator comprising a base sequence with 70% or more identity to the base sequence set forth in SEQ ID NO: 15, an insulator comprising a base sequence with 70% or more identity to the base sequence set forth in SEQ ID NO: 16, an insulator comprising a base sequence with 70% or more identity to the base sequence set forth in SEQ ID NO: 17, an insulator comprising a base sequence with 70% or more identity to the base sequence set forth in SEQ ID NO: 18, an insulator comprising a base sequence with 70% or more identity to the base sequence set forth in SEQ ID NO: 19, and the like. Among these, an insulator comprising a base sequence with 70% or more identity to the base sequence set forth in SEQ ID NO: 17 is particularly preferable from the viewpoint of further significantly improving genome editing efficiency.

In a preferred embodiment of the present invention, the sequence that binds to a nuclear translocation factor and/or an intranuclear structure is located downstream (on the 3' side) of the terminator.

In the polynucleotide of the present invention, the site-specific nuclease expression cassette preferably comprises (a) a promoter. The promoter is located on the 5' side of the site-specific nuclease coding sequence.

The promoter is not particularly limited as long as it is an upstream region of a gene that is involved in the initiation and promotion of gene transcription. The promoter typically comprises a transcription start site, a sequence upstream (on the 5' side) of the transcription start site, and, if necessary, a sequence downstream (on the 3' side) of the transcription start site. The promoter is, for example, any DNA region comprising a transcription start site in a DNA region of, for example, -10000 to +500, preferably -5000 to +200, more preferably -2000 to +150, wherein the base of the transcription start site of the gene is +1, the downstream (3' side) thereof is a positive value, and the upstream (5' side) thereof is 0 or a negative value. When a plurality of transcription start sites are present, the transcription start site with the highest amount of transcription can be selected. The length of the promoter (the number of base pairs: bp) is, for example, in the range of 100 to 10000 bp, preferably 200 to 5000 bp, and more preferably 500 to 3000 bp.

Preferably, the promoter may be a promoter derived from a plant gene or a plant virus. The promoter is located on the 5' side of the coding region of a gene and can be easily identified by sequence analysis. Preferred examples of the promoter include a CmYLCV promoter (specifically, for example, SEQ ID NO: 20 (= SEQ ID NO: 1) or a mutant sequence thereof), a UBQ promoter (specifically, for example, a lettuce ubiquitin promoter (SEQ ID NO: 21 or a mutant sequence thereof), a soybean ubiquitin promoter (SEQ ID NO: 22 or a mutant sequence thereof), a *Chrysanthemum morifolium* ubiquitin promoter (SEQ ID NO: 23, 24, 25, or a mutant sequence thereof), parsley ubiquitin promoter (SEQ ID NO: 26 or a mutant sequence thereof), or a *Perilla frutescens* ubiquitin promoter (SEQ ID NO: 27 or a mutant sequence thereof)), an EF-1α (preferably *Nicotiana benthamiana* EF-1α) promoter (specifically, for example, SEQ ID NO: 28 or a mutant sequence thereof), an RPS5A (preferably *Arabidopsis thaliana* RPS5A) promoter (specifically, for example, SEQ ID NO: 29 or a mutant sequence thereof), and the like.

The promoter may comprise one or more base sequence mutations (e.g., substitution, deletion, insertion, and addition) as long as the function is not significantly reduced. The promoter has, for example, 70% or more, preferably 80% or more, more preferably 90% or more, even more preferably 95% or more, still even more preferably 97% or more, and particularly preferably 99% or more identity to the base sequence of a wild-type promoter. The promoter also includes those that have been modified to enhance the function of the promoter, for example, by linking a plurality of elements or by increasing the length in bases of a specific sequence.

The promoter is preferably, for example a promoter comprising a base sequence with 70% or more identity to the base sequence set forth in SEQ ID NO: 20 (= SEQ ID NO: 1), a promoter comprising a base sequence with 70% or more identity to the base sequence set forth in any of SEQ ID NOs: 21 to 27, a promoter comprising a base sequence with 70% or more identity to the base sequence set forth in SEQ ID NO: 28, or a promoter comprising a base sequence with 70% or more identity to the base sequence set forth in SEQ ID NO: 29.

In one embodiment of the present invention, the promoter is preferably a shoot apex expression promoter. The shoot apex expression promoter is not particularly limited as long as it is a promoter that can be expressed in cells in a shoot apex.

The polynucleotide of the present invention preferably comprises a virus vector sequence. The virus vector sequence is not particularly limited as long as it comprises a sequence involved in virus replication.

The virus from which the virus vector sequence is derived is particularly preferably, for example, a geminivirus. The geminivirus is not particularly limited as long as it has a rolling circle DNA replication mechanism. Examples include viruses of the genus *Mastrevirus* of the family *Geminiviridae,* such as bean yellow dwarf virus (BeYDV), maize streak virus (MSV), wheat dwarf virus (WDV), tobacco yellow dwarf virus (TYDV), and chickpea chlorotic dwarf virus (CpCDV); viruses of the genus *Begomovirus* of the family *Geminiviridae,* such as tomato golden mosaic virus (TGMV), African cassava mosaic virus (ACMV), cabbage leaf curl virus (CaLCuV), tomato leaf curl virus (ToLCV), and Ageratum yellow vein virus (AYVV); viruses of the genus *Curtovirus* of the family *Geminiviridae,* such as beet severe curly top virus (BCTV); and the like.

In addition to virus vector sequences derived from the viruses described above, virus vector sequences derived from various plant viruses, such as tobacco mosaic virus, cucumber mosaic virus, apple latent spherical virus, barley stripe mosaic virus, bean pod mottle virus, beet curly top virus, brome mosaic virus, cabbage leaf curl virus, cotton leaf crumple virus, cymbidium mosaic virus, grapevine virus A, pea early browning virus, poplar mosaic virus, potato virus X, rice tungro bacilliform virus, satellite tobacco mosaic virus, tobacco curly shoot virus, and tobacco rattle virus, can also be used.

When the site-specific nuclease is a Cas protein, the polynucleotide of the present invention preferably further comprises a guide RNA expression cassette. The presence of the Cas protein expression cassette and the guide RNA expression cassette in the same molecule (polynucleotide) makes it possible to efficiently express the Cas protein and guide RNA in the same cell, thus further enhancing genome editing efficiency.

The guide RNA expression cassette comprises a guide RNA coding sequence.

The guide RNA coding sequence is not particularly limited as long as it is a base sequence encoding guide RNA.

The guide RNA is not particularly limited as long as it is used in the CRISPR/Cas system. For example, various guide RNAs capable of binding to the target site of genomic DNA and binding to Cas protein to guide the Cas protein to the target site of the genomic DNA can be used.

The phrase "target site" as used herein is a site on genomic DNA that is composed of a DNA strand (non-target strand) composed of a PAM (protospacer adjacent motif) sequence and a sequence of about 17 to 30 bases in length (preferably 18 to 25 bases in length, more preferably 19 to 22 bases in length, and particularly preferably 20 bases in length) adjacent to the 5'-side or 3' side of the PAM sequence; and a complementary DNA strand (target strand) thereof.

The PAM sequence varies depending on the type of Cas protein used. For example, the PAM sequence corresponding to the Cas9 protein derived from *S. pyogenes* (type II) is 5'-NGG, the PAM sequence corresponding to the Cas protein derived from S. *solfataricus* (type I-A) is 5'-CCN/5'-TCN, the PAM sequence corresponding to the Cas protein derived from *H. walsbyi* (type I-B) is 5'-TTC, the PAM sequence corresponding to the Cas protein derived from E. *coli* (type I-E) is 5'-ARG, the PAM sequence corresponding to the Cas protein derived from *E. coli* (type I-F) is 5'-CC, the PAM sequence corresponding to the Cas protein derived from *P. aeruginosa* (type I-F) is 5'-CC, the PAM sequence corresponding to the Cas9 protein derived from *S. thermophilus* (type II) is 5'-NNAGAA, the PAM sequence corresponding to the Cas9 protein derived from *S. agalactiae* (type II) is 5'-NGG, the PAM sequence corresponding to the Cas9 protein derived from *S. aureus* is 5'-NNGRRT, the PAM sequence corresponding to the Cas9 protein derived from *N. meningitidis* is 5'-NNNNGATT, and the PAM sequence corresponding to the Cas9 protein derived from *T. denticola* is 5'-NAAAAC. In the above PAM sequences, N indicates any base.

The guide RNA has a sequence involved in binding to the target site of genomic DNA (sometimes referred to as a "crRNA (CRISPR RNA) sequence"). When the crRNA sequence binds complementarily (preferably, in a complementary and specific manner) to a sequence of the target strand excluding the complementary sequence of the PAM sequence, the guide RNA can bind to the target site of genomic DNA.

Binding "complementarily" includes not only the case of binding based on perfect complementarity (A and T, and G and C), but also the case of binding based on complementarity to a degree that allows hybridization under stringent conditions. The stringent conditions can be determined based on the melting temperature (Tm) of the nucleic acid binding a complex or probe, as taught in Berger and Kimmel (1987, Guide to Molecular Cloning Techniques Methods in Enzymology, Vol. 152, Academic Press, San Diego, CA). For example, the washing conditions after hybridization generally include about "1×SSC, 0.1% SDS, 37°C." It is preferable that the hybridization state is maintained even when washing is performed under such conditions. Examples of the washing conditions also include, but are not limited to, stricter hybridization conditions of about "0.5×SSC, 0.1%SDS, 42°C" and even stricter hybridization conditions of about "0.1×SSC, 0.1%SDS, 65°C."

Specifically, in the crRNA sequence, a sequence that binds to the target sequence has, for example, 90% or more, preferably 95% or more, more preferably 98% or more, even more preferably 99% or more, and particularly preferably 100% identity to the non-target strand.

When the guide RNA has a sequence involved in binding to a Cas protein (sometimes referred to as a "tracrRNA (transactivating crRNA) sequence"), the tracrRNA sequence binds to the Cas protein to allow the Cas protein to be guided to the target site of genomic DNA.

The tracrRNA sequence is not particularly limited. The tracrRNA sequence is typically an RNA composed of a sequence of about 50 to 100 bases in length capable of forming multiple (generally three) stem loops, and the sequence varies depending on the type of Cas protein used. As the tracrRNA sequence, various known sequences can be used according to the type of Cas protein used.

The guide RNA generally contains the crRNA sequence and the tracrRNA sequence. The embodiment of the guide RNA may be a single-stranded RNA (sgRNA) containing the crRNA sequence and the tracrRNA sequence, or may be an RNA complex formed by complementarily binding an RNA containing the crRNA sequence and an RNA containing the tracrRNA sequence.

The polynucleotide of the present invention may comprise other base sequences in addition to the above. Examples of the other base sequences include drug resistance genes, reporter protein coding sequences, and expression cassettes thereof; protein tag coding sequences; signal sequence coding sequences; origin of replication; element sequences of binary vectors used in the Agrobacterium method (e.g., left border region and right border region); and the like.

Examples of drug resistance genes include chloramphenicol resistance genes, tetracycline resistance genes, neomycin resistance genes, erythromycin resistance genes, spectinomycin resistance genes, kanamycin resistance genes, hygromycin resistance genes, puromycin resistance genes, and the like.

The reporter protein is not particularly limited and is, for example, a luminescent (color-developing) protein, which emits light (develops a color) by reacting with a specific substrate, or a fluorescent protein, which emits fluorescence from excitation light. Examples of luminescent (color-developing) proteins include luciferase, β-galactosidase, chloramphenicol acetyltransferase, β-glucuronidase, and the like. Examples of fluorescent proteins include GFP, Azami-Green, ZsGreen, GFP2, HyPer, Sirius, BFP, CFP, Turquoise, Cyan, TFP1, YFP, Venus, ZsYellow, Banana, KusabiraOrange, RFP, DsRed, AsRed, Strawberry, Jred, KillerRed, Cherry, HcRed, mPlum, and the like. Examples of reporter proteins also includes a fusion protein of a luminescent (color-developing) protein or fluorescent protein, and another protein (e.g., a seed-specific protein); and a luminescent (color-developing) protein or fluorescent protein to which a known protein tag, known signal sequence, or the like is added.

Examples of protein tags include biotin, a His tag, a FLAG tag, a Halo tag, an MBP tag, an HA tag, a Myc tag, a V5 tag, a PA tag, and the like.

The polynucleotide of the present invention may be single-stranded or double-stranded. The polynucleotide of the present invention may also be linear or cyclic. Further, the polynucleotide of the present invention encompasses not only DNA and RNA, but also DNA and RNA that have undergone known chemical modifications, as shown below. In order to prevent degradation by hydrolytic enzymes such as nucleases, the phosphate residue (phosphate) of each nucleotide can be replaced by a chemically modified phosphate residue, such as phosphorothioate (PS), methylphosphonate, or phosphorodithionate. The hydroxyl group at position 2 of the sugar (ribose) of each ribonucleotide may be replaced by -OR (R is, for example, CH3 (2'-O-Me), CH₂CH₂OCH₃ (2'-O-MOE), CH₂CH₂NHC(NH)NH₂, CH₂CONHCH₃, CH₂CH₂CN, or the like) . Further, the base moiety (pyrimidine or purine) may be chemically modified; for example, a methyl group or a cationic functional group is introduced at position 5 of the pyrimidine base, or the carbonyl group at position 2 is replaced by thiocarbonyl. Other examples include, but are not limited to, modification of the phosphate moiety or hydroxyl moiety with biotin, an amino group, a lower alkyl amine group, an acetyl group, or the like. The term "polynucleotide" includes not only natural nucleic acids but also BNA (bridged nucleic acid), LNA (locked nucleic acid), and PNA (peptide nucleic acid).

In one embodiment, the polynucleotide of the present invention is a single-stranded polynucleotide (e.g., RNA). In another embodiment, the polynucleotide of the present invention is a double-stranded polynucleotide (e.g., a vector such as a binary vector).

The polynucleotide of the present invention may constitute a vector. The type of vector is not particularly limited. Examples include plasmid vectors; Agrobacterium vectors; plant virus vectors; and the like. In addition to vectors suitable for introduction into plants or plant cells, such as the above vectors, examples also include vectors for transferring the polynucleotide of the present invention to such vectors (e.g., Gateway (registered trademark) entry clone vectors). In one embodiment, the present invention relates to a vector (preferably a vector for plant genome editing) comprising the polynucleotide of the present invention.

The polynucleotide of the present invention can be easily produced according to a known genetic engineering method. For example, PCR, restriction enzyme cleavage, a DNA ligation technique, an in vitro transcription technique, and the like can be used for production. The polynucleotide of the present invention can also be easily produced by incorporating a necessary sequence (e.g., a site-specific nuclease coding sequence) into various commercially available plant virus vectors (e.g., a binary vector).

### 3. Composition for Plant Genome Editing, Kit for Plant Genome Editing, Plant Genome Editing Method, and Method for Producing Plant

The polynucleotide of the present invention can be used as a composition for plant genome editing or a kit for plant genome editing. Further, a plant genome editing method using the polynucleotide of the present invention and a method for producing a genome-edited plant using the polynucleotide of the present invention can be provided.

The composition for plant genome editing is not particularly limited as long as it comprises the polynucleotide of the present invention. The composition for plant genome editing may comprise other components. Examples of other components include, but are not particularly limited to, bases, carriers, solvents, dispersants, emulsifiers, buffers, stabilizers, excipients, binders, disintegrants, lubricants, thickeners, moisturizers, colorants, flavoring agents, chelating agents, and the like. The composition for plant genome editing may optionally comprise a polynucleotide comprising a guide RNA expression cassette. A donor polynucleotide may also be contained, if necessary.

The kit for plant genome editing is not particularly limited as long as it comprises the polynucleotide of the present invention, and may optionally suitably comprise other materials, reagents, instruments, etc. that are necessary for the implementation of the plant genome editing method of the present invention, such as nucleic acid introduction reagents and buffer solutions. As other materials necessary for the implementation of the plant genome editing method of the present invention, the kit for plant genome editing may optionally comprise a polynucleotide comprising a guide RNA expression cassette. A donor polynucleotide may also be contained, if necessary.

The plant genome editing method and the method for producing a plant include introducing the polynucleotide of the present invention into a plant.

The plant genome editing method and the method for producing a plant preferably include introducing a mitotic cell activating factor at the site of introduction of the polynucleotide of the present invention or controlling the expression of an endogenous mitotic cell activating factor at the site of introduction of the polynucleotide of the present invention. The mitotic cell activating factor is not particularly limited as long as it is capable of promoting proliferation of cells at the site of introduction of the polynucleotide of the present invention, promoting growth of the tissue into which the polynucleotide of the present invention is introduced, or changing the plane of cell division at the site of introduction. When the site of introduction is a shoot apex, examples of mitotic cell activating factors capable of promoting proliferation of cells or promoting growth of the tissue into which the polynucleotide of the present invention is introduced include a WUSCHEL-related homeobox transcription factor (preferably *Zea mays* WUS2, hereinafter referred to as "ZmWUS2") (the amino acid sequence being specifically, for example, SEQ ID NO: 11 or a mutant sequence thereof), isopentenyl transferase (ipt) (the amino acid sequence being specifically, for example, SEQ ID NO: 13 or a mutant sequence thereof), an NAC transcription factor (e.g., *Arabidopsis thaliana* CUC1) (the amino acid sequence being specifically, for example, SEQ ID NO: 30 or a mutant sequence thereof), a response regulator lacking N-terminal signal receiver domain (e.g., ARR1 in which the DDK signal receiver domain at the N-terminus of *Arabidopsis thaliana* is deleted) (the amino acid sequence being specifically, for example, SEQ ID NO: 31 or a mutant sequence thereof), Cyclin (e.g., *Arabidopsis thaliana* CycD2;1) (the amino acid sequence being specifically, for example, SEQ ID NO: 32 or a mutant sequence thereof), an MYB3R transcription factor lacking negative regulation domain (e.g., myb A2 in which amino acids at positions 631 to 1042 of *Nicotiana tabacum* were deleted) (the amino acid sequence being specifically, for example, SEQ ID NO: 33 or a mutant sequence thereof), E2F (e.g., *Arabidopsis thaliana* E2F3) (the amino acid sequence being specifically, for example, SEQ ID NO: 34 or a mutant sequence thereof), DP (e.g., *Arabidopsis thaliana* DPa) (the amino acid sequence being specifically, for example, SEQ ID NO: 35 or a mutant sequence thereof), and a bHLH transcription factor (e.g., *Arabidopsis thaliana* HEC1) (the amino acid sequence being specifically, for example, SEQ ID NO: 36 or a mutant sequence thereof), and examples of mitotic cell activating factors capable of changing the plane of cell division at the site of introduction include a protein phosphatase 2 inhibition factor (e.g., a inhibition factor of *Arabidopsis thaliana* PP2AC1-5 (for example, a polypeptide that dominantly inhibits the function of the protein; specifically, for example, SEQ ID NOs: 37 to 41 or mutant sequences thereof)), and a TONNEAU 2 inhibition factor (e.g., a inhibition factor of *Solanum lycopersicum* TON2 (the amino acid sequence being specifically, for example, SEQ ID NO: 42 or a mutant sequence thereof) (e.g., an RNAi vector that targets the protein for suppression of expression)). Examples further include combinations of the mitotic cell activating factors described above. Among these, from the viewpoint of further significantly improving genome editing efficiency, a combination of a WUSCHEL-related homeobox transcription factor (preferably ZmWUS2) and ipt, an NAC transcription factor (e.g., *Arabidopsis thaliana* CUC1), Cyclin (e.g., *Arabidopsis thaliana* CycD2;1), and a combination of E2F (e.g., *Arabidopsis thaliana* E2F3) and DP (e.g., *Arabidopsis thaliana* DPa) are preferable, a combination of WUSCHEL-related homeobox transcription factor (preferably ZmWUS2) and ipt, Cyclin (e.g., *Arabidopsis thaliana* CycD2;1), and a combination of E2F (e.g., *Arabidopsis thaliana* E2F3) and DP (e.g., *Arabidopsis thaliana* DPa) are more preferable, and a combination of WUSCHEL-related homeobox transcription factor (preferably ZmWUS2) and ipt is particularly preferable.

The mitotic cell activating factor expression cassette is not particularly limited as long as it contains the coding sequence in an expressible state or in a state in which the function of the target protein can be suppressed.

The mitotic cell activating factor and the factor targeted by the mitotic cell activating factor may comprise one or more amino acid sequence mutations (e.g., substitution, deletion, insertion, and addition) as long as the function is not significantly reduced. The amino acid sequence of the mitotic cell activating factor or its target factor has, for example, 70% or more, preferably 80% or more, more preferably 90% or more, even more preferably 95% or more, still even more preferably 97% or more, and particularly preferably 99% or more identity to a wild-type amino acid sequence.

The mitotic cell activating factor expression cassette preferably comprises a promoter, a terminator, and the like. In one embodiment, the expression cassette may have a configuration similar to that of the site-specific nuclease expression cassette of the polynucleotide of the present invention. The expression cassette may be incorporated into the polynucleotide of the present invention or may be incorporated into a polynucleotide that is a molecule different from the polynucleotide of the present invention.

The combined use of the mitotic cell activating factor and the CRISPR/Cas system improves genome editing efficiency. From this viewpoint, in one embodiment, the present invention relates to a plant genome editing method or method for producing a genome-edited plant that comprises introducing a site-specific nuclease expression cassette and a mitotic cell activating factor expression cassette into a plant. In one embodiment, the present invention also relates to a composition for plant genome editing, an agent for improving plant genome editing efficiency, and the like that comprise a polynucleotide comprising a mitotic cell activating factor expression cassette. To the configurations of the composition and agent, the configuration of the composition for plant genome editing comprising the polynucleotide of the present invention may be applied.

The method of introducing the polynucleotide of the present invention and the mitotic cell activating factor into a plant is not particularly limited and can be performed in accordance with a known method or an equivalent method.

Examples of sites of introduction in plants include shoot apices, flowers (in particular, egg cells, pollen, etc. in flowers), leaves, roots, and the like.

The introduction method is not particularly limited and can be suitably selected according to the type of substance to be introduced and the target of introduction. Examples of the introduction method include agrobacterium methods, such as floral dip and floral spray methods; particle bombardment method; virus-mediated nucleic acid delivery; and the like. Among these, for example, the particle bombardment method is preferable from the viewpoint of convenience, safety, etc.

Specific examples of the introduction method are described below.

As a first example of the introduction method (Introduction Example 1), introduction can be performed by a method comprising preparing a plasmid containing a promoter (e.g., a T7 promoter, T3 promoter, or 35S promoter) and the sequence of the polynucleotide of the present invention located downstream thereof (step a1). If the plasmid obtained in step a1 is a Ti plasmid containing a promoter that has the ability to activate transcription in plant cells, such as a 35S promoter, introduction can be performed by a method comprising introducing the plasmid obtained in step a1 into Agrobacterium, followed by culturing (step b1) and inoculating a plant with the culture liquid obtained in step b1 (by, for example, infiltration, toothpick inoculation, or aspiration injection) (step c1). Alternatively, introduction can be performed by a method comprising, for example, inoculating a plant with the plasmid obtained in step a1 (by, for example, friction inoculation or the particle bombardment method) (step c2), in place of the above-mentioned steps b1 and c1. Alternatively, introduction can be performed by a method comprising performing, for example, the leaf disk method, inflorescence infiltration method, or vacuum filtration method (step c3), in place of the above-mentioned step c1. A desired protein can be produced from a plasmid or T-DNA introduced into a plant according to these methods.

In the particle bombardment method, the surface of microparticles can be coated with nucleic acids encoding a nuclease gene targeting a gene of interest and a cell activating factor, and proteins, and the microparticles can be shot into target cells. As microparticles, metal microparticles are preferably used because they have a high specific gravity, which increases penetration into cells, and they are chemically inert and thus less likely to be harmful to living organisms. Among metal microparticles, gold particles, tungsten particles, and the like are particularly preferably used.

In the particle bombardment method, a gene of interest can be introduced into plant cells as follows. First, microparticles such as gold particles or tungsten particles are washed and sterilized, a nucleic acid (e.g., a recombinant vector, linear DNA, or RNA) or a protein, CaCl₂, and spermidine are added to the microparticles while being stirred using a vortex mixer or the like to coat the gold particles or tungsten particles with the DNA, RNA, or protein, and the particles are washed with ethanol or phosphate buffered saline.

The optimum particle size (diameter) of the microparticles can be suitably determined according to the type of target cells, the type of microparticles, gas pressure, etc.

The gold particles or tungsten particles are applied to a macrocarrier film as uniformly as possible using Pipetman or the like and then dried in a sterile environment such as a clean bench. The macrocarrier film and a plate on which target cells are placed are placed in a particle bombardment apparatus, and high-pressure helium gas is expelled from a gas acceleration tube toward the macrocarrier film. The macrocarrier film stops at a stopping plate, but the particles pass through the stopping plate and penetrate target cells placed below the stopping plate, thereby introducing the gene of interest.

The optimum distance between the stopping plate and the target cells can be suitably determined according to the type of target cells, the type and particle size of microparticles, gas pressure, etc.

The optimum gas pressure can be suitably determined according to the type of target cells, the type of microparticles, the distance between the target and the stopping plate, etc.

In the cells bombarded with the microparticles, the nucleic acid and/or the protein is released from the microparticles. The nucleic acid is transferred to the nucleus to initiate its expression so that the translated nuclease is transferred to the nucleus to cleave genomic DNA; then, mutation occurs during the process of repairing, thereby obtaining genome-edited cells. The protein is transferred to the nucleus (organelle) with a nuclear localization signal (which may be an organelle localization signal) and cleaves DNA, thereby enabling genome editing to occur. Genome editing can be applied not only to genomic genes of the nucleus, but also to genes of organelles (chloroplasts, mitochondria, and other organelles). In this case, each organelle localization signal may be linked to a nuclease and introduced, or a promoter that is expressed only in each organelle may be linked to a nuclease or the like.

After introduction, the plant is cultured for a certain period of time to express the site-specific nuclease in the plant, thereby enabling genome editing to occur. The culture period is, for example, 1 to 30 days.

The culture method is not particularly limited. For example, if the plant is a plant body growing in a growing environment or a part thereof, the growth continues as is. If the plant is a plant body isolated from a growing environment or a part thereof, or if the plant is separated from a plant body, the object is placed in an appropriate environment (for example, in a moist environment or in an environment in which a constant temperature is maintained) and cultured.

The presence of genome editing can be confirmed by using a nucleic acid obtained from the target product and following known methods, such as T7 Endonuclease I assay, CAPS (cleaved amplified polymorphic sequence) analysis, and a method for detecting a mutation in the base sequence around the target site for genome editing (around the cleavage site of site-specific nuclease) (e.g., a method that uses primers designed for the predicted mutation site and sequencing analysis).

Genome-edited plants can be produced by the plant genome editing method of the present invention.

Furthermore, according to the genome editing technique of the present invention, it is also expected to obtain genome-edited reproductive cells. By obtaining next-generation plants from the genome-edited reproductive cells, plants in which all cells have been genome-edited can be obtained.

### Examples

The present invention is described in detail below based on Examples; however, the present invention is not limited to these Examples.

### Example 1. Cas Expression Vector Construction 1

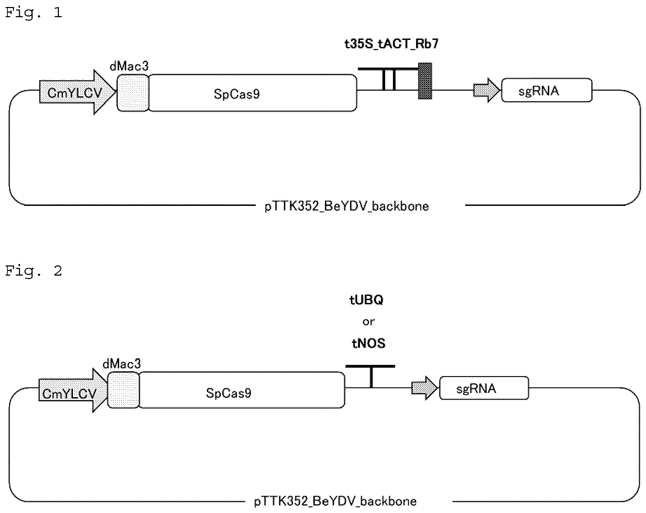

A Cas expression vector (Fig. 1) was obtained by inserting a Cas protein expression cassette and a guide RNA expression cassette into a pTTK352 binary vector comprising the sequence of a geminivirus vector (bean yellow dwarf virus (BeYDV)) .

The Cas expression vector was produced as follows. An artificially synthesized, BeYDV-derived sequence was inserted by seamless cloning between the left and right borders of the pKIR vector (binary) described in a previous report (Tsutsui et al. (2016), pKAMA-ITACHI Vectors for Highly Efficient CRISPR/Cas9-Mediated Gene Knockout in Arabidopsis thaliana, Plant and Cell Physiology, Vol. 58 (Issue 1), pp. 46-56). MP and CP derived from BeYDV were deleted, and the sequences encoding replicase proteins, as well as long intergenic regions (LIRs) and small intergenic regions (SIRs), were used.

The inserted Cas protein expression cassette was DNA in which the following items were arranged sequentially from the 5' end side:
(1) Cestrum yellow leaf curling virus (CtnYLCV) promoter (SEQ ID NO: 1);
(2) dMac3 sequence (SEQ ID NO: 2) or NtADH 5'UTR sequence (SEQ ID NO: 66);
(3) *Streptococcus pyogenes-derived* Cas9 (SpCas9) protein coding sequence having an epitope tag and a nuclear localization signal added thereto (SEQ ID NO: 3);
(4) 35S terminator of cauliflower mosaic virus (SEQ ID NO: 4);
(5) ACT3(ACTIN3) (NbACT3) terminator of *Nicotiana benthamiana* (SEQ ID NO: 5); and
(6) Rb7 scaffold/matrix attachment region (MAR) of *Nicotiana tabacum* (SEQ ID NO: 6).

The inserted guide RNA expression cassette was DNA in which the following items were arranged sequentially from the 5' end side:
(7) U6-26 promoter of *Arabidopsis thaliana* (SEQ ID NO: 7); and
(8) sgRNA and polyt sequences targeting sequences in the phytoene desaturase (NbPDS) gene of *Nicotiana benthamiana* (SEQ ID NO: 8).

The insertion method is described as follows. A Golden Gate method in which the method of Lampropoulos et al. (2013) was partly modified was used. The cassettes comprising the sequences 1 to 8 above were inserted by integrating the cassettes between the BeYDV-derived LIR and SIR sequences by performing a Golden Gate reaction with the NEB Golden Gate Assembly Kit. The reaction conditions were according to the recommended protocol of the kit. As a base sequence (4 bases) for linkage between the cassettes, ACCT was inserted into the immediate upstream of the cassette comprising the sequence 1 above, AACA was inserted between the cassette comprising the sequence 1 above and the cassette comprising the sequence 2 above, GGCT was inserted between the cassette comprising the sequence 2 above and the cassette comprising the sequence 3 above, ACTA was inserted between the cassette comprising the sequences 4 to 6 above and the cassette comprising the sequences 7 to 8 above, and GTAT was inserted into the immediate downstream of the cassette comprising the sequences 7 to 8 above. Further, a base sequence comprising TCAG + 46 bases + CTGC for linkage was inserted between the cassette comprising the sequence 3 above and the cassette comprising the sequences 4 to 6 above.

### Comparative Example 1: Cas Expression Vector Construction 2

A Cas expression vector (Fig. 2) was obtained in the same manner as in Example 1, except that a Cas protein expression cassette into which (4') a NOS terminator (SEQ ID NO: 9) was incorporated in place of (4), (5), and (6) was inserted as the Cas protein expression cassette.

### Comparative Example 2: Cas Expression Vector Construction 3

A Cas expression vector (Fig. 2) was obtained in the same manner as in Example 1, except that a Cas protein expression cassette into which (4") a UBQ terminator of *Arabidopsis thaliana* (SEQ ID NO: 43) was incorporated in place of (4), (5), and (6) was inserted as the Cas protein expression cassette.

### Test Example 1: Genome Editing Test 1

A genome editing test was conducted using each of the Cas expression vector of Example 1 and the Cas expression vectors of Comparative Examples 1 and 2, and the resulting genome editing efficiencies were compared. The details are described below.

### Preparation of Agrobacterium

Each of the Cas expression vector of Example 1 and the Cas expression vectors of Comparative Examples 1 and 2 was individually introduced into *Rhizobium radiobacter* strain GV3101 by electroporation, and the resulting product was spread on LB plates containing antibiotics, followed by culturing at 28°C for 3 days. Single colonies were pre-cultured overnight (28°C, 180 rpm) in 3 mL of LB liquid medium containing antibiotics. After addition of 7 mL of LB liquid medium and culturing for 2 hours (28°C, 180 rpm), the bacteria was collected. The collected bacteria were resuspended in an induction buffer (10 mM MES, 100 µM acetosyringone, pH of 5.5 with NaOH), and induction was performed for 2 to 3 hours. After the resulting product was resuspended in an infiltration buffer (5 mM MES, pH of 5.5 with NaOH), each agrobacterium liquid was adjusted such that OD=0.7 to obtain bacterial liquids for inoculation.

### Infiltration into Tobacco Leaves

After seeds of *Nicotiana benthamiana* for use were sterilized with Haiter, the seeds were spread on a seeding medium, allowed to incubate in the dark at 4°C for 4 days, and then allowed to grow for 1 week in a chamber at 25°C with a cycle of 16 hours of light and 8 hours of dark (16L8D). The seedlings were planted in culture soil obtained by mixing Hana-chan Culture Soil (Hanagokoro) and vermiculite at 1:1 and allowed to grow under the conditions of 25°C and 16L8D. Individuals obtained 2 to 3 weeks after transplantation were used for infiltration. Infiltration was performed from the abaxial side of the leaf by using a 1-mL syringe. After inoculation, the plant was kept moist to prevent drying and was allowed to grow in an environment at 26 to 30°C and 16L8D.

### Mutation Detection by T7EI Assay

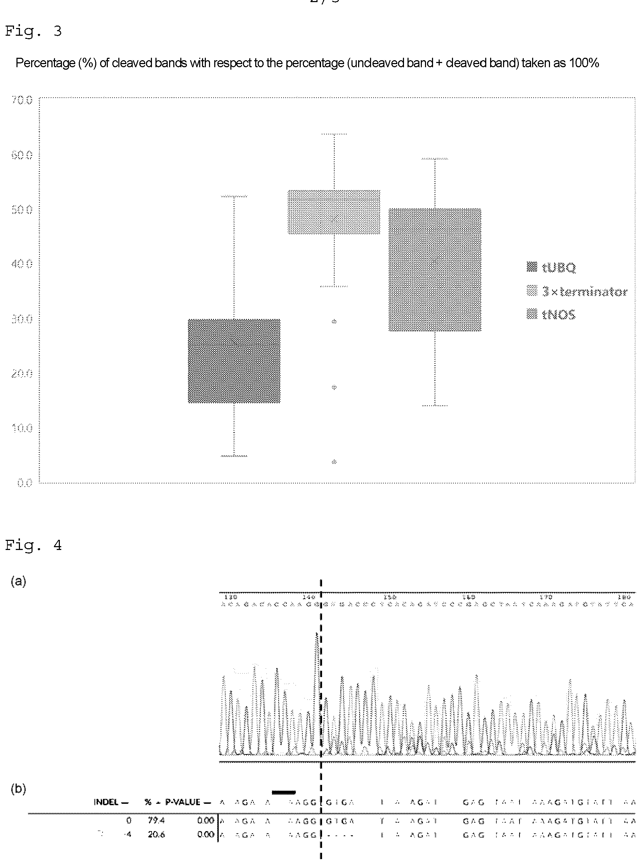

Seven days after inoculation, the inoculation sites were cut out with an 8-mm-diameter punch and crushed with an MB2200 Multi-beads shocker (Yasui Kikai Corporation), and total DNA was extracted using a Maxwell automated nucleic acid extraction system (Promega Corporation). Using the extracted DNA as a template, the DNA region containing the target sequence was PCR-amplified with KOD Fx Neo (TOYOBO Co., Ltd.). The PCR products were treated with T7 Endonuclease I (NEB), and the presence or absence of mutation introduction was detected with a MultiNA microchip electrophoresis system for DNA/RNA analysis (SHIMADZU Corporation). The presence of a cleaved band indicates the occurrence of genome editing. The percentage (%) of the cleaved bands was calculated with respect to the percentage (uncleaved band + cleaved band) taken as 100%, and was defined as genome editing efficiency. Fig. 3 and Table 1 show the results.

**Table 1**

| | tUBQ | 3×terminator | tNOS |
|---|---|---|---|
| Minimum value | 4.7 | 3.7 | 13.8 |
| First quartile | 14.4 | 45.4 | 27.6 |
| Second quartile | 25.1 | 51.6 | 46.3 |
| Third quartile | 29.7 | 53.3 | 49.9 |
| Maximum value | 52.2 | 63.6 | 59.1 |
| P value | 1.78E-06** | | 0.0116* |

| | | | |
|---|---|---|---|
| **p<0.01, *p<0.05 | | | |

When 35S + NbACT3 + Rb7 (3×terminator in Fig. 3 and Table 1) were used (the Cas expression vector of Example 1), mutation was detected with an efficiency of approximately 40 to 50%, and genome editing could occur with high efficiency in tobacco. Genome editing efficiency tended to be higher than when UBQ or NOS was used as a terminator.

### Example 2-1: Cas Expression Vector Construction 4

A Cas expression vector was obtained in the same manner as in Example 1, except that as the guide RNA expression cassette, a vector portion composed of eight tandemly linked expression cassettes in which sgRNA sequences targeting sequences in the genes of *Solanum lycopersicum* were placed under the control of the U6-26 promoter of *Arabidopsis thaliana* (the target genes of the sgRNAs in the expression cassettes were different: SlCCP1, SlGAD2, SlGAD3, SlSGR, SlLCY-E, SlBIc, SlLCY-B1, and SlLCY-B2, sequentially from the 5' side) was inserted.

### Example 2-2: Cas Expression Vector Construction 5

A Cas expression vector was obtained in the same manner as in Example 2-1, except that as the guide RNA expression cassette, a vector portion composed of four tandemly linked expression cassettes in which sgRNA sequences targeting sequences in the sg-5 gene of *Glycine max* were placed under the control of the U6-26 promoter of *Arabidopsis thaliana* was inserted.

### Example 2-3: Cas Expression Vector Construction 6

A Cas expression vector was obtained in the same manner as in Example 2-1, except that as the guide RNA expression cassette, a vector portion composed of six tandemly linked expression cassettes in which sgRNA sequences targeting sequences in the ADPG gene of *Perilla frutescens* were placed under the control of the U6-26 promoter of *Arabidopsis thaliana,* and a sequence encoding a kanamycin resistance gene were inserted.

### Comparative Example 3: Cas Expression Vector Construction 7

A Cas expression vector was obtained in the same manner as in Comparative Example 1, except that as the guide RNA expression cassette, a vector portion composed of eight tandemly linked expression cassettes in which sgRNA sequences targeting sequences in the genes of *Solanum lycopersicum* were placed under the control of the U6-26 promoter of *Arabidopsis thaliana* (the target genes of the sgRNAs in the expression cassettes were different: SlCCP1, SlGAD2, SlGAD3, SlSCR, SlLCY-E, SlBIc, SlLCY-B1, and SlLCY-B2, sequentially from the 5' side) was inserted.

### Reference Example 1: Expression Vector Construction

The following expression vectors were constructed as expression vectors to be introduced into a plant in Test Example 2.

An expression vector for a fluorescent protein (tdTomato) was constructed.

In addition, as mitotic cell activating factors, an expression vector for *Zea mays* WUS2 (WUSCHEL 2) gene (coding sequence: SEQ ID NO: 10; amino acid sequence: SEQ ID NO: 11), and an expression vector for ipt (isopentenyl transferase) gene (cytokinin biosynthesis gene) (coding sequence: SEQ ID NO: 12; amino acid sequence: SEQ ID NO: 13) were prepared. These two expression vectors were constructed in the same manner as in the Cas expression vector of Example 1, except that a ZmWUS2 coding sequence or ipt coding sequence was used in place of (3) the SpCas9 coding sequence, and a spacer sequence was used in place of (7) and (8) the guide RNA expression cassette.

### Test Example 2-1: Genome Editing Test 2-1

Genome editing was performed using each of the Cas expression vector of Example 2-1 and the Cas expression vector of Comparative Example 3, and the resulting genome editing efficiencies were compared. The genome editing efficiencies with and without the mitotic cell activating factors of Reference Example 1 were also compared. The details are described below.

### Aseptic Cultivation of Tomato

The commercially available tomato cultivar "Home Momotaro" (Takii & Co., Ltd.) was used. Seeds were washed with 70% ethanol, sterilized with Haiter, allowed to absorb water overnight, and spread on a seeding medium (MS, 0.5% sucrose, 0.4% gellan gum).

### Removal of Shoot Apex

The operation was aseptically performed on Table KOACH T500-F (Koken). The aerial part was cut out with a scalpel to about 2 to 3 cm and placed on filter paper (Advantec) moistened with sterile water. The subsequent operation was performed under a stereomicroscope (OLYMPUS SZX10). The leaf primordia were removed using a 1-mL syringe (Terumo) and a 26G injection needle (Terumo) so that the shoot apex was not damaged. The shoot apex was placed on a medium (MS basal salt, 3% sucrose, 150 mg/L cefotaxime, 0.01 µM gibberellic acid3, 0.01 µM kinetin, 0.6% Phytagel) in a 6-cm dish (Iwaki) with the shoot apex portion facing straight up and allowed to incubate overnight.

### Particle Bombardment

Particle bombardment was performed using PDS-1000/He (Bio-Rad) with a modified protocol. Gold particles (InBio) with a diameter of 0.6 um were washed with 70% ethanol, rinsed three times with sterile water, and suspended in 50% glycerol. 5 µg of a plasmid was mixed with 3 mg of the gold particles, together with 0.1M spermidine and 2.5M CaCl₂. The plasmids used in test groups and their molar ratios are as described below.
- Test group 1: Cas expression vector (Example 2-1):tdTomato expression vector = 1:1
- Test group 2: Cas expression vector (Comparative Example 3):tdTomato expression vector = 1:1
- Test group 3: Cas expression vector (Example 2-1):tdTomato expression vector:ZmWUS2 expression vector (Reference Example 1):ipt expression vector (Reference Example 1) = 1:1:1:1

Subsequently, the resulting gold particles were washed with 70% ethanol and then suspended in 100% ethanol. The prepared gold particles were uniformly spread over the center of a macrocarrier while being suspended, and were dried. Two 650 psi rupture disks were inserted into a retaining cap, and the macrocarrier with the gold particles and a stopping screen were inserted into a microcarrier launch assembly, and they were placed in PDS-1000. The dish with the shoot apex was placed in the PDS-1000, followed by bombardment. The dish with the shoot apex was then wrapped in aluminum foil and allowed to incubate overnight.

### Confirmation of Introduction

On the day after the bombardment, the number of bright spots of tdTomato on each shoot apex was counted using a BZ-X810 fluorescent microscope (KEYENCE), and the shoot apices were transferred to a fresh medium (MS basal salt, 3% sucrose, 150 mg/L cefotaxime, 0.01 µM gibberellic acid3, 0.01 µM kinetin, 0.3% Phytagel).

### Transplantation

Seven days after the bombardment, the shoot apices were observed, and shoot apices with five or more bright spots and alive were transferred to growth media (MS, 3% sucrose, 150 mg/L cefotaxime, 0.01 µM gibberellic acid3, 0.01 µM kinetin, 0.3% Phytagel in ECO2box) and allowed to grow in the conditions of 25°C and 18L6D.

### Detection of Genome Editing by T7EI Assay

The newly expanded 5th leaf was sampled and grinded with an MB2200 Multi-beads shocker (Yasui Kikai Corporation), and total DNA was extracted using a Maxwell automated nucleic acid extraction system (Promega Corporation). Using the extracted DNA as a template, the fragment containing the target sequence was PCR-amplified with KOD Fx Neo (TOYOBO Co., Ltd.). The PCR products were treated with T7 Endonuclease I (NEB), and the presence or absence of mutation introduction was detected with a MultiNA microchip electrophoresis system for DNA/RNA analysis (SHIMADZU Corporation). Table 2 shows the results.

**Table 2**

| | Number analyzed | Number of mutant individuals for each gene | | | | | | | | Number of individuals in which any of the eight genes was edited | Genome editing efficiency (%) |
|---|---|---|---|---|---|---|---|---|---|---|---|
| | | SIGAD2 | SIGAD3 | SISGR | SIBIc | SILCY-E | SILCY-B1 | SILCY-B2 | SIGGP | | |
| 35S terminator +ACT terminator +Rb7 MAR | 118 | 0 | 2 | 3 | 6 | 2 | 0 | 2 | 0 | 7 | 5.9 |
| NOS terminator | 83 | 0 | 1 | 2 | 2 | 0 | 0 | 0 | 0 | 2 | 2.4 |
| 35S terminator +ACT terminator +Rb7 MAR +ZmWUS2 +ipt | 99 | 3 | 3 | 6 | 6 | 2 | 1 | 3 | 1 | 9 | 9.1 |

When the NOS terminator was used (the Cas expression vector of Comparative Example 3), mutation introduction was detected at 2.4%. When the 35S terminator + NbACT3 terminator + Rb7 MAR was used (the Cas expression vector of Example 2-1), mutation introduction was detected at 5.1%, which was 2.1 times higher than when the NOS terminator was used. When the ZmWUS2 expression vector and ipt expression vector (the expression vectors of Reference Example 1) were shot together with the genome editing vector, the mutation introduction percentage was 9.1%, which was 1.8 times higher than when ZmWUS2 or ipt was not introduced.

### Detection of Genome Editing in Next Generation

The above-obtained individuals into which mutation had been introduced were allowed to grow to obtain next-generation seeds. The obtained seeds were sown, and total DNA was extracted from each cotyledon of multiple individuals. Using the extracted DNA as a template, the fragment containing the target sequence on the SGR gene was PCR-amplified using KOD Fx Neo (TOYOBO Co., Ltd.), and analyzed for the presence or absence of mutation by Sanger sequencing. As a result, "mutant homozygotes," in which mutation was 100% introduced, "mutant heterozygotes," in which mutation was 50% introduced, and "no mutation," in which no mutation was introduced, were separately detected, and it was confirmed that the mutation was transmitted to the next-generation individuals.

### Test Example 2-2: Genome Editing Test 2-2

Genome editing was performed using the Cas expression vector of Example 2-2. The details are described below.

### Aseptic Cultivation of Soybean

The commercially available soybean cultivar "Fukuyutaka" was used. Seeds sterilized by chlorine gas sterilization was placed on a moist PROWIPE to absorb water.

### Removal of Shoot Apex

Shoot apices were removed in the same manner as in Test Example 2-1.

### Particle Bombardment

Particle bombardment was performed in the same manner as in Test Example 2-1 (test group 3).

### Confirmation of Introduction

On the day after the bombardment, the number of bright spots of tdTomato on each shoot apex was counted using a BZ-X810 fluorescent microscope (KEYENCE), and the shoot apices were transferred to a fresh medium (MS basal salt, 3% sucrose, 25 mg/L meropenem, 0.01 µM gibberellic acid3, 0.01 µM kinetin, 0.3% Phytagel).

### Transplantation

Seven days after the bombardment, the shoot apices were observed, and shoot apices with one or more bright spots and alive were transferred to a growth medium (MS, 3% sucrose, 25 mg/L meropeneme, 0.01 µM gibberellic acid3, 0.01 µM kinetin, 0.3% Phytagel in ECO2box) and allowed to grow in the conditions of 25°C and 18L6D.

### Mutation Detection by Sanger Sequencing

Total DNA was extracted from the newly expanded 5th leaf. Using the extracted DNA as a template, the fragment containing the target sequence was PCR-amplified using KOD Fx Neo (TOYOBO Co., Ltd.), and sequence data was obtained by Sanger sequencing (Fig. 4a). Based on the obtained sequence data, analysis was performed for the presence or absence of mutation using the analysis software "DECODR." As a result, a four-base deletion was detected (Fig. 4b).

### Detection of Genome Editing in Next Generation

The individuals in which mutation introduction was confirmed in the above-mentioned present generation were allowed to grow, and whether the mutation was introduced in the resulting next-generation seeds was analyzed in the same manner as in Test Example 2-1. As a result, the same mutation as the four-base deletion detected in the present generation was detected, confirming that the mutation was transmitted to the next-generation individuals.

### Test Example 2-3: Genome Editing Test 2-3

Genome editing was performed using the Cas expression vector of Example 2-3. The details are described below.

### Preparation of Perilla Plant

*Perilla frutescens* "Shirakawa Okute" was used. After absorbing water overnight, the seeds were sown in the soil and cultivated.

### Removal of Shoot Apex

Shoot apices were removed in the same manner as in Test Example 2-1.

### Particle Bombardment

Particle bombardment was performed in the same manner as in Test Example 2-1 (test group 3).

### Confirmation of Introduction

On the day after the bombardment, the number of bright spots of tdTomato on each shoot apex was counted using a BZ-X810 fluorescent microscope (KEYENCE), and the shoot apices were transferred to a fresh medium (MS basal salt, 3% sucrose, 25 mg/L meropeneme, 0.01 µM gibberellic acid3, 0.01 µM kinetin, 0.3% Phytagel).

### Transplantation

Seven days after the bombardment, the shoot apices were observed, and shoot apices with five or more bright spots and alive were transferred to a growth medium (MS, 3% sucrose, 25 mg/L meropeneme, 0.01 µM gibberellic acid3, 0.01 µM kinetin, 0.3% Phytagel in ECO2box).

### Mutation Detection by CAPS (Cleaved Amplified Polymorphic Sequence)

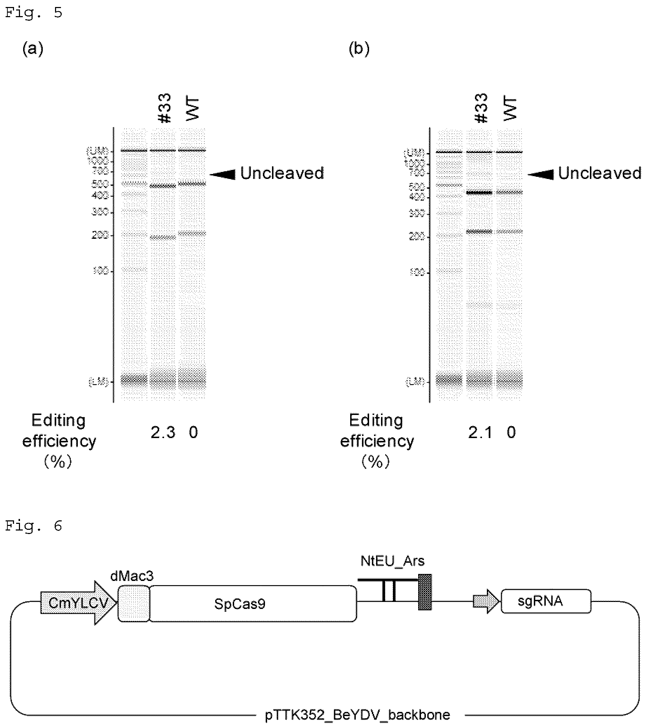

Total DNA was extracted from the newly expanded 5th leaf. Using the extracted DNA as a template, the fragment containing the target sequence was PCR-amplified using KOD Fx Neo (TOYOBO Co., Ltd.). The PCR products were column-purified, treated with BsrGI (NEB) and SspI (NEB), and analyzed for the presence or absence of mutation introduction using a MultiNA microchip electrophoresis system for DNA/RNA analysis (SHIMADZU Corporation). The presence of an uncleaved band indicates the occurrence of genome editing. The percentage (%) of the uncleaved bands was calculated with respect to the percentage (uncleaved band + cleaved band) taken as 100% and was defined as genome editing efficiency. Fig. 5(a) shows the results of treatment with BsrGI, and Fig. 5(b) shows the results of treatment with SspI. CAPS analysis shows that about 2% mutation introduction was detected.

### Comparative Example 4: Cas Expression Vector Construction 8

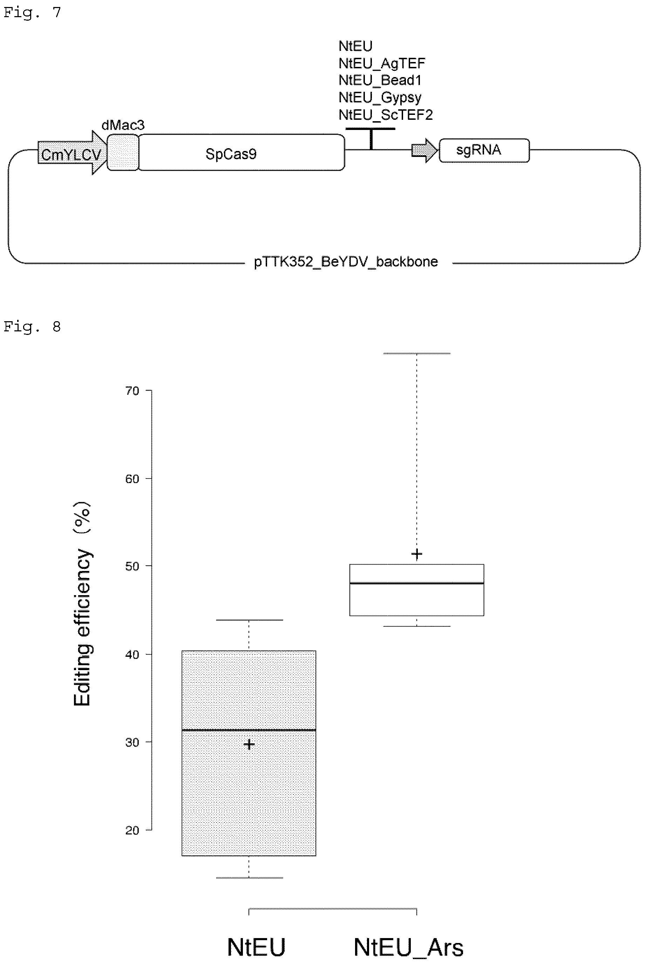

A Cas expression vector (Fig. 7) was obtained in the same manner as in Example 1, except that a Cas protein expression cassette in which (4"") an extensin terminator of *Nicotiana tabacum* (SEQ ID NO: 14) was incorporated in place of (4), (5), and (6) in the Cas protein expression cassette was inserted.

### Example 3: Cas Expression Vector Construction 9

A Cas expression vector (Fig. 7) was obtained in the same manner as in Example 1, except that a Cas protein expression cassette in which (4"") an extensin terminator of *Nicotiana tabacum* (SEQ ID NO: 14) was incorporated in place of (4) and (5), and a TEF (translation elongation factor) insulator of A. *gossypii* (SEQ ID NO: 18) was incorporated in place of (6) in the Cas protein expression cassette was inserted.

### Example 4: Cas Expression Vector Construction 10

A Cas expression vector (Fig. 6) was obtained in the same manner as in Example 1, except that a Cas protein expression cassette in which (4"") an extensin terminator of *Nicotiana tabacum* (SEQ ID NO: 14) was incorporated in place of (4) and (5), and a sea urchin Ars (arylsulfatase) insulator (SEQ ID NO: 17) was incorporated in place of (6) in the Cas protein expression cassette was inserted.

### Example 5: Cas Expression Vector Construction 11

A Cas expression vector (Fig. 7) was obtained in the same manner as in Example 1, except that a Cas protein expression cassette in which (4"") an extensin terminator of *Nicotiana tabacum* (SEQ ID NO: 14) was incorporated in place of (4) and (5), and a human Bead1 (blocking element alpha/delta-1 of the human T-cell receptor α/ δ locus) insulator (SEQ ID NO: 16) was incorporated in place of (6) in the Cas protein expression cassette was inserted.

### Example 6: Cas Expression Vector Construction 12

A Cas expression vector (Fig. 7) was obtained in the same manner as in Example 1, except that a Cas protein expression cassette in which (4"") an extensin terminator of *Nicotiana tabacum* (SEQ ID NO: 14) was incorporated in place of (4) and (5), and a Drosophila Gypsy insulator (SEQ ID NO: 15) was incorporated in place of (6) in the Cas protein expression cassette was inserted.

### Example 7: Cas Expression Vector Construction 13

A Cas expression vector (Fig. 7) was obtained in the same manner as in Example 1, except that a Cas protein expression cassette in which (4"") an extensin terminator of *Nicotiana tabacum* (SEQ ID NO: 14) was incorporated in place of (4) and (5), and a yeast TEF2 (translation elongation factor 2) insulator (SEQ ID NO: 19) was incorporated in place of (6) in the Cas protein expression cassette was inserted.

### Test Example 3: Genome Editing Test 3

Genome editing was performed using each of the Cas expression vectors of Examples 3 to 7 and the Cas expression vector of Comparative Example 4, and the mutation introduction percentages were compared. The test was performed in the same manner as in Test Example 1.

Fig. 8 shows the results of Example 4 and Comparative Example 4 as representative examples. When the sea urchin Ars insulator was fused to the extensin terminator of *Nicotiana tabacum,* a mutation introduction percentage of 44% or more was detected, and genome editing could be induced with high efficiency in tobacco. When the sea urchin Ars insulator was used (Example 4), genome editing efficiency was much higher than in Examples 3 and 5 to 7, which used other insulators.

### Example 8: MAD7 Expression Vector Construction

A MAD7 expression vector was obtained by inserting a MAD7 protein expression cassette and a guide RNA expression cassette into a pTTK352 binary vector comprising the sequence of a geminivirus vector (bean yellow dwarf virus (BeYDV)).

The MAD7 expression vector was produced as follows. An artificially synthesized, BeYDV-derived sequence was inserted by seamless cloning between the left and right borders of the pKIR vector (binary) described in a previous report (Tsutsui et al. (2016), pKAMA-ITACHI Vectors for Highly Efficient CRISPR/Cas9-Mediated Gene Knockout in Arabidopsis thaliana, Plant and Cell Physiology, Vol. 58 (Issue 1), pp. 46-56). MP and CP derived from BeYDV were deleted, and the sequences encoding replicase proteins, as well as long intergenic regions (LIRs) and small intergenic regions (SIRs), were used.

The inserted MAD7 protein expression cassette was DNA in which the following items were arranged sequentially from the 5' end side:
(1) Cestrum yellow leaf curling virus (CmYLCV) promoter (SEQ ID NO: 1);
(2) dMac3 sequence (SEQ ID NO: 2);
(3) MAD7 protein coding sequence having an epitope tag and a nuclear localization signal added thereto (SEQ ID NO: 44);
(4) 35S terminator of cauliflower mosaic virus (SEQ ID NO: 4);
(5) ACT3(ACTIN3) (NbACT3) terminator of *Nicotiana benthamiana* (SEQ ID NO: 5); and
(6) Rb7 scaffold/matrix attachment region (MAR) of *Nicotiana tabacum* (SEQ ID NO: 6).

The inserted guide RNA expression cassette was DNA in which the following items were arranged sequentially from the 5' end side:
(7) 35S promoter of cauliflower mosaic virus (SEQ ID NO: 45);
(8) hammerhead (HH) ribozyme (SEQ ID NO: 46);
(9) direct repeat sequence (SEQ ID NO: 47);
(10) sequence from *Nicotiana benthamiana* XT1 gene as a spacer (SEQ ID NO: 48);
(11) hepatitis delta virus (HDV) ribozyme (SEQ ID NO: 49); and
(12) Nos terminator (SEQ ID NO: 50).

The insertion method is described as follows. A Golden Gate method in which the method of Lampropoulos et al. (2013) was partly modified was used. The cassettes comprising the sequences above were inserted by integrating the cassettes between the BeYDV-derived LIR and SIR sequences by performing a Golden Gate reaction with the NEB Golden Gate Assembly Kit. The reaction conditions were according to the recommended protocol of the kit. As a base sequence (4 bases) for linkage between the cassettes, ACCT was inserted into the immediate upstream of the cassette comprising the sequence 1 above, AACA was inserted between the cassette comprising the sequence 1 above and the cassette comprising the sequence 2 above, GGCT was inserted between the cassette comprising the sequence 2 above and the cassette comprising the sequence 3 above, ACTA was inserted between the cassette comprising the sequences 4 to 6 above and the cassette comprising the sequences 7 to 12 above, and GTAT was inserted into the immediate downstream of the cassette comprising the sequences 7 to 12 above. Further, a base sequence comprising TCAG + 46 bases + CTGC for linkage was inserted between the cassette comprising the sequence 3 above and the cassette comprising the sequences 4 to 6 above.

### Test Example 4: Genome Editing Test 4

A genome editing test was performed using the effector expression vector of Example 8 and a silencing suppressor expression vector (pDGB3alpha2_35S:P19:Tnos; document: Sarrion-Perdigones A, Vazquez-Vilar M, Palaci J, Castelijns B, Forment J, Ziarsolo P, Blanca J, Granell A, Orzaez D. (2013) GoldenBraid 2.0: a comprehensive DNA assembly framework for plant synthetic biology. Plant Physiol. 162(3): 1618-1631), and the mutation introduction percentage was analyzed. The details are described below.

### Preparation of Agrobacterium

The effector expression vector of Example 8 and the silencing suppressor expression vector were introduced into *Rhizobium radiobacter* strain GV3101 by electroporation, and the resulting product was spread on LB plates containing antibiotics, followed by culturing at 28°C for 2 to 3 days. Single colonies were pre-cultured overnight (28°C, 180 rpm) in 3 mL of LB liquid medium containing antibiotics. After addition of 7 mL of LB liquid medium and culturing for 2 hours (28°C, 180 rpm), the bacteria was collected. The collected bacteria were resuspended in an induction buffer (10 mM MES, 100 µM acetosyringone, pH of 5.5 with NaOH), and induction was performed for 2 to 3 hours. After the resulting product was resuspended in an infiltration buffer (5 mM MES, pH of 5.5 with NaOH), each agrobacterium liquid was adjusted such that OD=0.7 and mixed in equal amounts to obtain a bacterial liquid for inoculation.

### Infiltration into Tobacco Leaves

After seeds of *Nicotiana benthamiana* for use were sterilized with Haiter, the seeds were spread on a seeding medium, allowed to incubate in the dark at 4°C for 4 days, and then allowed to grow for 1 week in a chamber at 25°C with a cycle of 16 hours of light and 8 hours of dark (16L8D). The seedlings were planted in culture soil obtained by mixing Hana-chan Culture Soil (Hanagokoro) and vermiculite at 1:1 and allowed to grow under the conditions of 26 to 30°C and 16L8D. Individuals obtained 2 to 3 weeks after transplantation were used for infiltration. Infiltration was performed from the underside of the leaf by using a 1-mL syringe. After inoculation, the plant was kept moist to prevent drying and was allowed to grow in an environment at 26 to 30°C and 16L8D.

### Mutation Detection by CAPS (Cleaved Amplified Polymorphic Sequence)

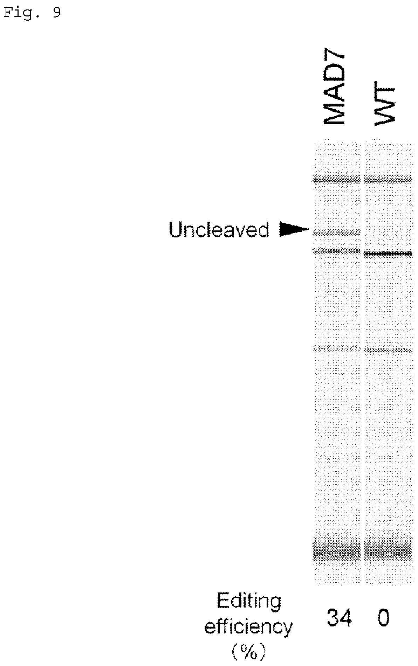

Seven days after inoculation, the inoculation sites were cut out with an 8-mm-diameter punch and grinded with an MB2200 Multi-beads shocker (Yasui Kikai Corporation), and total DNA was extracted using a Maxwell automated nucleic acid extraction system (Promega Corporation). Using the extracted DNA as a template, the DNA region containing the target sequence was PCR-amplified with KOD Fx Neo (TOYOBO Co., Ltd.). The PCR products were column-purified (Macherey-Nagel) and then treated with EcoRI (NEB), and the presence or absence of mutation introduction was detected with a MultiNA microchip electrophoresis system for DNA/RNA analysis (SHIMADZU Corporation). The presence of an uncleaved band indicates the occurrence of genome editing. The percentage (%) of the uncleaved bands was calculated with respect to the percentage (uncleaved band + cleaved band) taken as 100%, and was defined as genome editing efficiency. Fig. 9 shows the results.

### Results

CAPS analysis shows that a mutation introduction percentage of 34% was detected even when the genome editing tool MAD7 was used. Thus, in addition to Cas9, genome editing can also be induced with high efficiency when MAD7 was used, and thus, it is believed that the vector can be applied to various genome editing tools.

### Reference Example 2: Expression Vector Construction

The following expression vectors were constructed as expression vectors to be introduced into a plant in Test Example 5.

A Cas expression vector was constructed in the same manner as in Example 1, except that as the guide RNA expression cassette, a vector portion composed of seven tandemly linked expression cassettes in which sgRNA sequences targeting the FAD7 gene sequence of *Solanum lycopersicum* were placed under the control of the U6-26 promoter of *Arabidopsis thaliana* was inserted.

As mitotic cell activating factors, an expression vector for *Arabidopsis thaliana* CUC1 (CUP-SHAPED COTYLEDON 1) gene (NAC transcription factor) (coding sequence: SEQ ID NO: 51; amino acid sequence: SEQ ID NO: 30), an expression vector for *Arabidopsis thaliana* ARR1 (Arabidopsis Response Regulator 1) gene in which the DDK signal receiver domain at the N-terminus was deleted (coding sequence: SEQ ID NO: 52; amino acid sequence: SEQ ID NO: 31), an expression vector for *Arabidopsis thaliana* CycD2;1 gene (coding sequence: SEQ ID NO: 53; amino acid sequence: SEQ ID NO: 32), an expression vector for *Nicotiana tabacum* myb A2 gene in which amino acids at positions 631 to 1042 were deleted (MYB3R transcription factor) (coding sequence: SEQ ID NO: 54; amino acid sequence: SEQ ID NO: 33), an expression vector for *Arabidopsis thaliana* E2F3 gene (coding sequence: SEQ ID NO: 55; amino acid sequence: SEQ ID NO: 34), an expression vector for *Arabidopsis thaliana* DPa gene (coding sequence: SEQ ID NO: 56; amino acid sequence: SEQ ID NO: 35), an expression vector for *Arabidopsis thaliana* HEC1 gene (bHLH transcription factor) (coding sequence: SEQ ID NO: 57; amino acid sequence: SEQ ID NO: 36), and an expression vector for *Arabidopsis thaliana* PP2AC1-5 gene in which amino acid mutation was introduced so as to be dominant negative (protein phosphatase 2)(coding sequence: SEQ ID NOs: 58 to 62; amino acid sequence: SEQ ID NOs: 37 to 41) were prepared.

The above-mentioned 12 expression vectors were constructed in the same manner as in the Cas expression vector of Example 1, except that each mitotic cell activating factor coding sequence was inserted in place of the SpCas9 coding sequence, and a spacer sequence was inserted in place of the guide RNA expression cassette.

In addition, an expression suppression (RNAi) vector for *Solanum lycopersicum* TON2 (TONNEAU 2) gene (coding sequence: SEQ ID NO: 63; amino acid sequence: SEQ ID NO: 42) was prepared.

The TON2 expression suppression vector was constructed in the same manner as in the Cas expression vector of Example 1, except that a target sequence of TON2 gene (SEQ ID NO: 64), a base sequence (ACGA) for linkage, a pdk intron (SEQ ID NO: 65), a base sequence (TCGT) for linkage, and a reverse complementary sequence of the target sequence of TON2 gene were inserted in this order from the 5' end side in place of the SpCas9 coding sequence in order to form ihpRNA (intron-containing hairpin RNA) targeting the TON2 coding sequence, and a spacer sequence was inserted in place of the guide RNA expression cassette.

### Test Example 5: Genome Editing Test 5

The genome editing efficiencies with and without the mitotic cell activating factors of Reference Example 2 were compared. The details are described below.

### Aseptic Cultivation of Tomato

Aseptic cultivation of tomato was performed in the same manner as in Test Example 2-1.

### Removal of Shoot Apex

Shoot apices were removed in the same manner as in Test Example 2-1.

### Particle Bombardment

Particle bombardment was performed in the same manner as in Test Example 2-1.

The plasmids used in test groups and their molar ratios are as described below.
- Test group 1: Cas expression vector:tdTomato expression vector = 1:1
- Test group 2: Cas expression vector:tdTomato expression vector: ZmWUS2 expression vector (Reference Example 1):ipt expression vector (Reference Example 1) = 1:1:1:1
- Test group 3: Cas expression vector:tdTomato expression vector: AtCUC1 expression vector = 1:1:1
- Test group 4: Cas expression vector:tdTomato expression vector: AtARR1ΔDDK expression vector = 1:1:1
- Test group 5: Cas expression vector:tdTomato expression vector:AtCycD2;1 expression vector = 1:1:1
- Test group 6: Cas expression vector:tdTomato expression vector:NtMYBA2Δ631-1042 expression vector = 1:1:1
- Test group 7: Cas expression vector:tdTomato expression vector:AtE2F3 expression vector: Dpa expression vector = 1:1:1:1
- Test group 8: Cas expression vector:tdTomato expression vector:AtHEC1 expression vector = 1:1:1
- Test group 9: Cas expression vector:tdTomato expression vector:ZmWUS2 expression vector (Reference Example 1):ipt expression vector (Reference Example 1):AtCUC1 expression vector:AtARR1ΔDDK expression vector:AtCycD2;1 expression vector:NtMYBA2Δ631-1042 expression vector:AtE2F3 expression vector:Dpa expression vector = 1:1:1:1:1:1:1:1:1:1
- Test group 10: Cas expression vector:tdTomato expression vector:PP2AC1 expression vector:PP2AC2 expression vector:PP2AC3 expression vector:PP2AC4 expression vector:PP2AC5 expression vector = 1:1:1:1:1:1:1
- Test group 11: Cas expression vector:tdTomato expression vector:TON2 expression suppression vector = 1:1:1

### Confirmation of Introduction

Confirmation of introduction was performed in the same manner as in Test Example 2-1.

### Transplantation

Transplantation was performed in the same manner as in Test Example 2-1.

### Detection of Genome Editing by Sanger Sequencing

The newly expanded 5th leaf was sampled and grinded in extraction buffer (1M KCl, 10 mM EDTA, 100 mM Tris-HCl with HCl) using a pestle until the buffer turned green. The extract was mixed well in a vortex and kept warm at 95°C for 10 minutes. After being kept warm, the extract was mixed well again in a vortex and centrifuged, and the supernatant was used as a template for PCR.

The fragment containing the target sequence was PCR-amplified using KOD Fx Neo (TOYOBO Co., Ltd.). The PCR products were treated with Exo-CIP Rapid PCR Cleanup Kit (NEB) and then analyzed for the presence or absence of mutation introduction by Sanger sequencing. Table 3 shows the results of test group 1, test group 2 (ZmWUS2+ipt), test group 3 (AtCUC1), test group 5 (AtCyc2;1), test group 7 (AtE2F3+Dpa), and test group 11 (TON2).

### Results

**Table 3**

| Mitotic cell activating factor | Average number of bright spots on shoot apex | Number analyzed | Number of edited individuals | Genome editing efficiency (%) |
|---|---|---|---|---|
| None | 2.5 | 35 | 2 | 5.7% |
| ZmWUS2+ipt | 2.1 | 36 | 5 | 13.9% |
| AtCUC1 | 2.4 | 27 | 2 | 7.4% |
| AtCycD2;1 | 3.1 | 29 | 3 | 10.3% |
| AtE2F3+Dpa | 4.2 | 30 | 3 | 10.0% |
| TON2 | 2.4 | 34 | 3 | 8.8% |

A comparison of the mutation introduction percentage (5.7%) when no mitotic cell activating factor was introduced and those when each factor was introduced revealed that the mutation introduction percentage was increased when each of ZmWUS2+ipt, AtCUC1, AtCyc2;1, AtE2F3+Dpa, and TON2 was individually used (13.9%, 7.4%, 10.3%, 10.0%, and 8.8%, respectively). In these cases, the mutation introduction percentages were much higher than when other mitotic cell activating factors were introduced.

### Detection of Genome Editing in Next Generation

The individuals in which mutation introduction was confirmed in the 5th leaf were allowed to grow, and the resulting next-generation seeds were sown. In germinated cotyledons, detection of genome editing was performed using the same method as in the detection in the 5th leaf described above. As a result, mutation was also detected in the next-generation individuals.

### Example 9: LbCpf1 Expression Vector Construction

An LbCpf1 expression vector was obtained by inserting an LbCpf1 protein expression cassette and a guide RNA expression cassette into a pTTK352 binary vector containing a sequence of a geminivirus vector (bean yellow dwarf virus (BeYDV)).

The LbCpf1 expression vector was constructed in the same manner as in Example 8, except that an LbCpf1 protein coding sequence having an epitope tag and a nuclear localization signal added thereto (SEQ ID NO: 67) was used in (3), and a direct repeat sequence (SEQ ID NO: 68) for LbCpf1 was used in (9).

### Test Example 6. Genome Editing Test 6

A genome editing test was performed in a plant using the effector expression vector of Example 9 and a silencing suppressor expression vector (pDGB3alpha2_35S:P19:Tnos; document: Sarrion-Perdigones A, Vazquez-Vilar M, Palaci J, Castelijns B, Forment J, Ziarsolo P, Blanca J, Granell A, Orzaez D. (2013) GoldenBraid 2.0: a comprehensive DNA assembly framework for plant synthetic biology. Plant Physiol. 162(3): 1618-1631), and the mutation introduction percentage was analyzed. Specifically, the test was performed in the same manner as in Test Example 4.

### Results

CAPS analysis shows that mutation introduction was also detected when the genome editing tool LbCpf1 was used, as in MAD7. Thus, in addition to Cas9 and MAD7, genome editing can also be induced when LbCpf1 was used, and the vector can be applied to various genome editing tools.

## Claims

1. A polynucleotide comprising a site-specific nuclease expression cassette and a sequence that binds to a nuclear translocation factor and/or an intranuclear structure, the site-specific nuclease expression cassette comprising:
(b) a site-specific nuclease sequence; and
(c) a 3'UTR comprising a terminator.

2. The polynucleotide according to claim 1, wherein the sequence that binds to a nuclear translocation factor and/or an intranuclear structure is an insulator.

3. The polynucleotide according to claim 1 or 2, wherein the sequence that binds to a nuclear translocation factor and/or an intranuclear structure is contained in the 3'UTR.

4. The polynucleotide according to any one of claims 1 to 3, wherein the sequence that binds to a nuclear translocation factor and/or an intranuclear structure is an insulator other than a scaffold/matrix attachment region.

5. The polynucleotide according to claim 4, wherein the insulator is at least one member selected from the group consisting of an Ars insulator, a Gypsy insulator, a Bead1 insulator, a TEF insulator, and a TEF2 insulator.

6. The polynucleotide according to claim 5, wherein the insulator is an Ars insulator.

7. The polynucleotide according to any one of claims 1 to 3, wherein the sequence that binds to a nuclear translocation factor and/or an intranuclear structure is a scaffold/matrix attachment region.

8. The polynucleotide according to any one of claims 1 to 7, wherein the terminator is a linked body of at least two terminators.

9. The polynucleotide according to any one of claims 1 to 8, wherein the sequence that binds to a nuclear translocation factor and/or an intranuclear structure is located in the 3'UTR and downstream of the terminator.

10. The polynucleotide according to any one of claims 1 to 9, wherein the terminator is at least one member selected from the group consisting of a 35S terminator, an ACT3 terminator, and an extensin terminator.

11. The polynucleotide according to any one of claims 1 to 10, wherein the site-specific nuclease expression cassette comprises (a) a promoter.

12. The polynucleotide according to claim 11, wherein the promoter is a shoot apex expression promoter.

13. The polynucleotide according to claim 11 or 12, wherein the promoter is a CmYLCV promoter, a UBQ promoter, an EF-1α promoter, or an RPS5A promoter.

14. The polynucleotide according to any one of claims 1 to 13, which comprises a virus vector sequence.

15. The polynucleotide according to claim 14, wherein the virus vector sequence is a geminivirus vector sequence.

16. The polynucleotide according to claim 14 or 15, wherein the virus vector sequence is a BeYDV sequence.

17. The polynucleotide according to any one of claims 1 to 16, further comprising a guide RNA expression cassette.

18. A vector for plant genome editing, comprising the polynucleotide of any one of claims 1 to 17.

19. A kit for plant genome editing, comprising the polynucleotide of any one of claims 1 to 17.

20. A plant genome editing method comprising introducing the polynucleotide of any one of claims 1 to 17 into a plant.

21. The plant genome editing method according to claim 20, further comprising introducing a mitotic cell activating factor into the site of introduction of the polynucleotide.

22. A method for producing a genome-edited plant, comprising introducing the polynucleotide of any one of claims 1 to 17 into a plant.
